(19) Europäisches Patentamt
European Patent Office
Office européen des brevets

(11) EP 0 810 993 B1

(12) FASCICULE DE BREVET EUROPEEN

(45) Date de publication et mention de la délivrance du brevet:
**28.11.2001 Bulletin 2001/48**

(21) Numéro de dépôt: **96904161.5**

(22) Date de dépôt: **19.02.1996**

(51) Int Cl.$^7$: **C07C 327/22**, C07C 327/32, C07C 327/34, C07C 381/04, C07C 391/02, C07B 37/04, C07B 37/02, C07B 45/06, C07C 319/14, C07C 17/32, C07C 17/272

(86) Numéro de dépôt international:
**PCT/FR96/00264**

(87) Numéro de publication internationale:
**WO 96/26185 (29.08.1996 Gazette 1996/39)**

# (54) REACTIF, COMPOSE ET PROCEDE POUR LA PERFLUOROALCYLATION DE NUCLEOPHILE, AINSI QUE LES DERIVES OBTENUS

VERFAHREN ZUR HERSTELLUNG VON PERHALOALKYLIERTEN UND/ODER PERHALOACYLIERTEN VERBINDUNGEN SOWIE PERHALOALKYLIERUNGSMITTEL UND/ODER PERHALOACYLIERUNGSMITTEL

REAGENT, COMPOUND AND METHOD FOR NUCLEOPHILE PERFLUOROALKYLATION, AND RESULTING DERIVATIVES

(84) Etats contractants désignés:
**AT BE CH DE DK ES FR GB GR IE IT LI LU MC NL PT SE**

(30) Priorité: **24.02.1995 FR 9502157**
**15.09.1995 FR 9510937**

(43) Date de publication de la demande:
**10.12.1997 Bulletin 1997/50**

(73) Titulaire: **RHODIA CHIMIE**
**92408 Courbevoie Cédex (FR)**

(72) Inventeurs:
- **LANGLOIS, Bernard**
  **F-69006 Lyon (FR)**
- **ROQUES, Nicolas**
  **F-34200 Sète (FR)**
- **WAKSELMAN, Claude**
  **75016 Paris (FR)**
- **TORDEUX, Marc**
  **F-92330 Sceaux (FR)**
- **FORAT, Gérard**
  **F-69003 Lyon (FR)**

(74) Mandataire: **Ricalens, François et al**
**Rhodia Services,**
**Direction de la Propriété Industrielle,**
**40, rue de la Haie-Coq**
**93306 Aubervilliers Cedex (FR)**

(56) Documents cités:
**EP-A- 0 307 519** **EP-A- 0 458 684**

- **JOURNAL OF THE CHEMICAL SOCIETY, PERKIN TRANSACTIONS 1, no. 3, 1978, LETCHWORTH, GB, pages 202-209, XP002004616 C.J. BROOKES, ET AL.: "Reactions of fluoroalkyl radicals generated electrochemically. Part 1. Additions of trifluoromethyl radicals to olefinic and acetylinic bonds"**
- **PHOSPHORUS, SULFUR, SILICON AND THE RELATED ELEMENTS, vol. 59, no. 1-4, 1991, LONDON, GB, pages 169-172, XP002004617 J.-L. CLAVEL, ET AL.: "Trifluoromethylation of organic disulphides with sodium trifluoromethane- sulphinate under oxidative conditions: synthesis of trifluoromethyl thioethers"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

(Cont. page suivante)

- **CANADIAN JOURNAL OF CHEMISTRY, vol. 52, no. 17, 1 Septembre 1974, OTTAWA, CA, pages 3113-3118, XP000572119 E. BOCK, ET AL.: "Study of d-orbital effects in esters of trifluorothiolacetic acid. A comparison of evidence from dipole moment and kinetic data"**
- **JOURNAL OF FLUORINE CHEMISTRY, vol. 1, no. 3, Janvier 1972, LAUSANNE, CH, pages 295-307, XP000572174 P. WEEKS, ET AL.: "Chemistry of trifluorothiolacetic acid and its derivatives. II"**
- **JOURNAL OF FLUORINE CHEMISTRY, vol. 8, no. 1, 1976, LAUSANNE, CH, pages 101-104, XP000572176 R.N. HASZELDINE, ET AL.: "The reactions of N,N-bistrifluoromethylaminotrifluoro-methanesulphonate with nucleophiles"**
- **INORGANIC CHEMISTRY, vol. 12, no. 8, Août 1973, WASHINGTON, DC, US, pages 1896-1899, XP002004618 R.A. DE MARCO, ET AL.: "Reactions of the difluoroamine-potassium fluoride adduct, HNF2.KF, with sulphinyl and perfluoro- alkylsulphinylfluorides. Preparation of perfluoroalkyl perfluoroalkylsulphinates"**
- **BULLETIN OF THE ACADEMY OF SCIENCES OF THE USSR, DIVISION OF CHEMICAL SCIENCE, vol. 34, no. 6, part 2, Juin 1985, NEW YORK, US, pages 1281-1283, XP002004619 V.M. BZHEZOVSKII, ET AL.: "13C nmr and X-ray fluorescence spectra of a series of benzyl sulphides"**
- **JOURNAL OF FLUORINE CHEMISTRY, vol. 35, no. 3, Mars 1987, LAUSANNE, CH, pages 523-530, XP002004620 T. NGUYEN, ET AL.: "Perfluorothioalkanoyl halides. Preparation from sulphides"**

## Description

La présente invention a pour objet une technique de perfluoroalcoylation et notamment un réactif, un composé et un procédé pour la perfluoroalcoylation de nucléophile, ainsi que les dérivés ainsi obtenus. Elle concerne plus particulièrement l'utilisation de la coupure homolytique de certains esters chalcogénés avec des chalcogènes relativement lourds, c'est-à-dire de rang atomique au moins égal à celui du soufre.
Ainsi, le domaine de l'invention est celui de la synthèse de composés perhalogénoalcoylés et/ou acylés :

- soit par greffage de radicaux perhalogénoalcoylés et/ou acylés sur des substrats, au moins partiellement organique et de natures variées, lesdits radicaux étant fournis par des agents de perhalogénoalcoylation et/ou acylation,

- soit par autotransformation directe d'agents de perhalogénoalcoylation.

La présente invention concerne ainsi un procédé d'obtention de composés perhalogénés, en particulier perhalogénoalcoylés et/ou acylés, ainsi que de thioéthers perhalogénoalcoylés et/ou acylés.
L'halogène, plus précisément mais non limitativement considéré, est le fluor, en raison du grand intérêt qui existe à l'utiliser comme substituant dans de nombreux produits industriels tels que les colorants, les polymères, les composés pharmaceutiques et agrochimiques. Il est, en effet, bien connu que le fluor est un inducteur de lipophilie sur les molécules biologiquement actives.
En outre, son électronégativité et sa taille relativement petite sont des facteurs positifs au regard :

- de l'absorption de la lumière par les colorants,

- de la stabilité, de la solubilité et des propriétés mécaniques et électriques des polymères,

- et des propriétés biologiques de certaines molécules pharmaceutiques et/ou agrochimiques.

Compte tenu de l'intérêt scientifique et technique du substituant trifluorométhyle, notamment dans les domaines pharmaceutique et agrochimique, de nombreuses méthodes de trifluorométhylation ont déjà été proposées.
Parmi celles-ci, la trifluorométhylation directe, selon un mécanisme électrophile, nucléophile et/ou radicalaire, est la plus digne d'intérêt sur le plan technique et industriel.
Les techniques de trifluorométhylation par voie radicalaire présentent l'avantage de permettre souvent d'opérer dans des conditions très douces.
Certes, par cette voie, on connaît la perfluoroalcoylation à l'aide d'iodures de perfluoroalcoyles tels que $CF_3I$. Ces composés réagissent sur divers substrats par voie thermique, sous irradiation ou en présence d'une quantité catalytique d'un initiateur radicalaire, tel que le peroxyde de benzoyle ou l'AzobisIsoButyroNitrile (AIBN). Les iodures de perfluoroalcoyles sont des produits onéreux et peu disponibles au niveau industriel.
En outre, les réactions concernées sont susceptibles d'engendrer comme sous-produits, des produits iodés réputés toxiques, du type $CF_3 - CH_2 - CH_2 - I$.
Les dérivés des acides perfluoroalcanesulfoniques qui conviennent également pour une voie radicalaire, sont eux aussi coûteux et encore peu disponibles à l'échelle industrielle.
Les dérivés du nitrosotrifluorométhane peuvent également être utilisés, mais souffrent de l'inconvénient d'être toxiques et de synthèse dangereuse.
Les acides perfluoroalcanoïques et leurs dérivés tels que l'acide trifluoroacétique auraient éventuellement pu être employés comme agents de perhalogénoalcoylation. Mais il s'est avéré qu'ils sont difficilement transformables en radical $\cdot CF_3$. L'oxydation électrochimique mise en oeuvre à cette fin nécessite, en effet, des potentiels très élevés et des excès importants de ce réactif. La faisabilité industrielle du procédé faisant intervenir ces composés n'est donc pas acquise.
Le trifluorométhanethiol ($CF_3SH$) et le chlorure de trifluorométhane sulfényle $CF_3SCl$ sont connus comme étant des agents réactifs pour la trifluorométhylthiolation. Mais, ils présentent l'inconvénient d'être gazeux et très toxiques.
En pratique, l'agent de trifluorométhylation le plus courant, est le bromotrifluorométhane ($CF_3Br$). Ce produit industriel est employé comme agent d'extinction des feux, notamment dans les avions et les salles d'ordinateurs. Il est l'une des plus importantes sources actuelles de radicaux trifluorométhyles.
Le $CF_3Br$ peut être réduit monoélectroniquement pour donner $\cdot CF_3$, radical susceptible d'être piégé, soit par des substrats nucléophiles, réducteurs et initiateurs tels que des thiolates, des thiophénates, des sélènophénates, des carbanions stabilisés ou des énamines, soit par un réducteur du type $SO_2^{-\cdot}$.
A titre d'exemple d'une transformation, selon la susdite technique, de substrats disulfures donnant naissance

à des alcoyles ou des aryl sulfures trifluorométhylés, on se référera à l'article CLAVEL et al. : *Phosphorus, Sulfur & Silicon*, 1991, vol. 59, p. 129-132. L'article de WAKSELMAN et al. : *J. Chem. Soc., Chem. Commun*, 1984, p. 793-794, donne une illustration de la trifluorométhylation de thiophénols à l'aide de $CF_3$ Br.

Alternativement, $\cdot CF_3$ peut être engendré à l'aide d'un oxydant du type hydroperoxyde de tertiobutyle (t-BuOOH) et de trifluorométhanesulfinate de sodium ($CF_3SO_2$ Na), lui-même obtenu à partir de bromotrifluorométhane et de dithionite de sodium. Dans ces conditions, les disulfures sont transformés en trifluorométhylthioéthers ($RSCF_3$) (cf. CLAVEL & al. : *Phosphorus, Sulfur & Silicon,* 1991, vol. 59, pages 169-172,), les composés aromatiques sont trifluorométhylés (cf. LANGLOIS & al : *Tetrahedron letters*, 1991, vol. 32, n° 51, p. 7525-7528) et les esters d'énols conduisent à des -trifluorométhylcétones (cf. LANGLOIS & al. : *Tetrahedron letters,* 1992, vol. 33, n° 10, p. 1291-1294).

Le problème lié à ces techniques connues, utilisant le bromotrifluorométhane comme source de $\cdot CF_3$, tient à la difficulté de manipulation de ce gaz. De surcroît, c'est un produit qui est voué à l'interdiction par les réglementations internationales sur l'environnement, en raison de ses effets nocifs supposés sur l'environnement, notamment en raison de l'effet de serre qu'il induirait, et des effets hypothétiques sur la couche d'ozone.

Il va donc en découler un besoin important en agents de trifluorométhylation radicalaire, qui soient commodes à mettre en oeuvre, non toxiques, facilement disponibles et peu coûteux.

Ainsi, l'un des objectifs de la présente invention est de fournir un procédé d'obtention de composés perhalogénés, en particulier perhalogénoalcoylés et/ou acylés, dans lequel on a recours à des substituts avantageux du bromotrifluorométhane.

Un autre but de la présente invention est de fournir un réactif du type précédent qui permette de greffer un radical perfluoré sur un électrophile

Un autre but de la présente invention est de fournir des composés qui soient susceptibles d'être utilisés pour le réactif et/ou dans le procédé qui précèdent.

Un autre but de la présente invention est de fournir des dérivés qui soient susceptibles d'être obtenus par les réactifs et procédé du type précédent.

La Demanderesse est parvenue à mettre au point un tel réactif, qui se caractérise par le fait qu'il il comporte pour addition successive ou simultanée :

→ un composé de formule (I):

$$R_f\text{-}M(X)(Z)_n\text{-}Y\text{-}R\ ;$$

avec R représentant un radical carboné, avantageusement d'au plus 15 de préférence 10 atomes de carbone, de préférence choisi parmi les alcoyles les aryles, les acyles, les thioacyles [tel que les hydrocarbylchalcogénylacyles (et notamment carbonyles) (ici les chalcogènes préférés sont les soufre et l'oxygène), notamment les aryloxyacyles, les alcoyloxyacyles et en particulier les aryloxycarbonyles, les alcoyloxycarbonyles les aryloxythiocarbonyles, les alcoyloxythiocarbonyles ou leur homologues lorsque les oxygènes sont remplacés totalement ou partiellement par le soufre] ;

avec $R_f$, présentant avantageusement au plus 20 atomes de carbone, de préférence au plus 15 atomes de carbone, représentant un radical de formule (II) :

$$[R_2\text{-}(C\Xi_2)_p\text{-}][R_1\text{-}(C\Xi_2)_m\text{-}]CF\text{-}$$

où $R_1$ et $R_2$, semblables ou différents, représentent un atome d'halogène léger, fluor ou de chlore, de préférence fluor , un radical carboné

où m est zéro ou un entier choisi dans l'intervalle fermé de 0 à 12, avantageusement au plus égal à 8, de préférence au plus égal à 6 ;

où p est zéro ou un entier choisi dans l'intervalle fermé de 0 à 12, avantageusement au plus égal à 6, de préférence au plus égal à 4 ;

où les Ξ semblables ou différents représentent des radicaux perhalogénés, avantageusement perfluorés, des atomes de chlore ou de préférence de fluor ; avec la condition que lorsque m et/ou p sont égaux à zéro, $R_1$ et/ou $R_2$

sont électroattracteurs, avantageusement un atome de fluor ou de chlore, , de préférence un atome de fluor.

avec n représentant zéro ou 1 ;

avec M représentant un métalloïde choisi parmi le carbone et les chalcogènes de rang supérieur à l'oxygène ;

avec X, Y, Z semblables ou différents représentant un chalcogène avec la condition que X, Y et Z ne soient pas tous oxygène quand n est différent de zéro;

→ une source de radicaux.

Lorsque M est carbone n est égal à zéro.
Certains des composés de formules (I) ont été décrits mais leur propriété de perfluoroalcoylation ne l'ont pas été.
Ainsi des thioloester de bas poids moléculaire ont été décrits dans les articles suivants :

- E.BOCK ET AL. ,CANADIAN JOURNAL OF CHEMISTRY, vol.52, n°17,3113-3118,1974;
- P. WEEKS ET AL., JOURNAL OF FLUORINE CHEMISTRY, vol. 1, n°3,295-307,1972;
- T.NGUYEN,ET AL JOURNAL OF FLUORINE CHEMISTRY,vol.35,n"3,523-530,1987 ;

Cependant que des perfluorométhanethiosulfonate et perfluoroéthanethiosulfonate de perfluoroalcoyle était décrits dans l'article de R. A. DE MARCO ET AL., INORGANIC CHEMISTRY, vol. 12, n"8,1896-1899,1973.
Ainsi R est avantageusement un radical aliphatique ou alicyclique, de préférence alcoyle, y compris aralcoyle, aralcényle aralcynyle, alcényle et alcynyle, et aryle, , linéaire ou ramifié, substitué ou non.
R peut être également un radical de formule $R_f$-M(X)(Z)$_n$- où $R_f$ M, X, Y, Z et n ont la même signification qu'exposée précédemment, sans cela signifie que la molécule soit nécessairement symétrique.
Rappelons que l'expression "hydrocarbylchalcogényle" est un radical de structure $R_a$-Y"- où $R_a$- est un radical hydrocarboné, c'est-à-dire comportant au moins de l'hydrogène et du carbone et dont l'atome porteur de la liaison (ici avec Y") est un carbone, et où Y" est un chalcogène (oxygène, soufre, sélénium, tellure). $R_a$ est avantageusement un alcoyle [éventuellement substitué, et notamment halogéné (y compris perhalogéné et notamment perfluoré)] ou un aryle éventuellement substitué.
Selon la présente invention, si l'on désire éviter les réactions parasites, il est hautement souhaitable que R soit choisi de manière que le radical R' ne soit que peu ou pas stable ; ainsi les benzyles, les alcoyles tertiaires et tout radical dont le radical libre correspondant est aussi, ou plus, stables que ceux précédemment cités, sont à éviter.
On peut utiliser les composés de formule (I) de telle sorte et dans de telle conditions qu'ils, et notamment les thioloesters de formule (I) puissent engendrer des radicaux libres.
Ledit générateur de radicaux est une source actinique. Le procédé selon l'invention consiste à faire réagir le thioloester de formule (I) avec un substrat insaturé au moins partiellement organique, de préférence sous irradiation à une longueur d'onde comprise entre 200 et 800 nanomètres ; ledit générateur de radicaux est alors une source de rayonnement de longueur d'onde compris entre 200 et 800 nm et, de préférence, entre 210 et 600 nm (nanomètres).
Pour obtenir les meilleurs résultats il est conseillé de se placer à la longueur d'onde correspondant au maximum d'absorption de la fonction "chromophore -M(X)(Z)$_n$-Y-
**[0034]** Ledit générateur de radicaux est aussi une source chimique de radicaux libres. Ainsi que cela a déjà été mentionné précédemment, on peut utiliser les composés de formule (I) de telle sorte et dans de telle conditions qu'ils, et notamment les thioloesters de formule (III) puissent engendrer des radicaux libres.
Lesdits mécanismes font intervenir un initiateur, qui est de nature chimique [par exemple les peroxydes, qu'ils soient symétriques, mixtes et/ou hydroperoxydes, d'acyle(s) (dont le plus courant est celui de benzoyle), peroxyde d'alcoyle (en général tertiaire, dont les plus usités sont ceux des tertiobutyle), ou dérivés azoïques (tels que azobisisobutyronitrile = AIBN)].avantageusement en présence de stannane (notamment hydrogenostannane) et/ou de silanes (notamment hydrogénosilane) ; les stannanes et notamment les distannanes (tels que $Bu_3SnSnBu_3$), peuvent être utilisé seuls.
Avantageusement le réactif comporte en outre un solvant. le solvant peut également être constitué d'un excès d'un des réactifs.
Lorsque l'on utilise une initiation actinique, pour éviter que l'énergie de rayonnement hν initiant la réaction radicalaire, ne soit absorbée par le solvant au détriment du thioloester (I), il importe de choisir le solvant de manière qu'il possède une transmittance supérieure ou égale à 50 %, de préférence à 70 % et, plus préférentiellement encore, à 90 %, à la longueur d'onde de travail.
Aussi, conformément à l'invention quand il y initiation actinique, le solvant est, de préférence, sélectionné parmi les composés suivants : acétonitrile ; éthanol ; butanol ; dichlorométhane ; cyclohexane ; cyclopentane ; dichloro-1,2 éthane ; diméthyl-2,2 butane ; n-heptane ; n-hexane ; méthanol ; méthyl-2 butane ; iso-octane ; n-pentane ; propanol-2 ; trichloro-1,2,2trifluoroéthane ; trifluoro-2,2,2éthanol ou un mélange de ceux-ci.
Lorsque l'on utilise d'autre sorte d'initiation que l'actinique, il convient d'utiliser des solvants qui soient inertes vis à vis des composés (I) comme dans tous les cas mais aussi vis à vis des initiateurs qu'ils soient physiques (radiation, température pour I thermolyse radicaux issus des initiateurs chimiques). Si l'on s'intéresse maintenant aux composés

de formule (I)

$$R_f\text{-}M(X)(Z)_n\text{-}Y\text{-}R\ ;$$

Il est souhaitable qu'au moins une, de préférence deux conditions compatibles ci-après soient remplies :

- Y soit sélénium, souffre et tellure avantageusement choisi parmi le sélénium et le soufre, de préférence un soufre ;
- Z est choisi parmi l'oxygène et le soufre, de préférence un oxygène
- X est choisi parmi l'oxygène et le soufre, de préférence l'oxygène ;
- M est un atome de carbone, n est zéro, X est oxygène et Y est soufre ou sélénium ;
- M est un atome de soufre ou de sélénium, n est un, X est oxygène et Y est soufre ou sélénium.

Ainsi, au cours de l'étude qui a mené à la présente invention, Il a été trouvé que certain des composés qui peuvent être mis en oeuvre dans le réactif décrit ci-dessus, n'avaient jamais été décrits avaient dû être synthétisés et étaient nouveaux.

Ainsi, un autre but de la présente invention est de fournir un composé du type précédent. Ainsi qu'un procédé de synthèse de ces composés. Ces buts et d'autres qui apparaitront par la suite sont atteints au moyen de composé (s) de formule (I)

avec (i) R représentant un radical carboné, préferablement hydrocarboné, avantageusement d'au plus 15, preferablement 10 atomes de carbone, de préférence choisi parmi les acyles et les thisacyles,
(ii) ou R radical carboné avantageusement d'au plus 15, de préférence 10 atomes de carbone, de préférence choisi parmi les alcoyles, les aryles les acyles, y compris les hydrocarbylchalcogénylcarnonyles et les thioacyles, y compris hydrocarbylchalcogénylthiocarsonyle et $R_f$ ne comporte pas plus de trois carbones
avec $R_f$ représentant un radical de fomlule :

$$[R_2\text{-}(C\Xi_2)_p\text{-}][R_1\text{-}(C\Xi_2)_m\text{-}]CF\text{-}$$

où $R_1$ et $R_2$, semblables ou différents, représentent un atome d'halogène léger, fluor ou de chlore, de préférence fluor , un radical carboné
où m est zéro ou un entier compris entre 0 et 12 ;
où p est zéro ou un entier compris entre 0 et 12 ;
où les Ξ semblables ou différents représentent des radicaux perhalogénés,
avantageusement perfluorés, des atomes de chlore ou de préférence de fluor ;
avec la condition que lorsque m et/ou p sont égaux à zéro. $R_1$ et/ou $R_2$ sont est électroattracteurs avantageusement un atome de fluor ou de chlore, de préférence un atome de fluor.
avec n représentant zéro ou 1 ;
avec M représentant un métalloide choisi parmi le carbone et les chalcogènes de rang supérieur à l'oxygène ;
avec (i) Y représentant un soufre ou un sélénium ;

avec X, Z semblables ou différents représentant un chalcogène ou (ii) x, y, z chalcogènes $R_1$ est un radical carboné choisi parmi les alcoyles et les aryles.
$R_f$ est un radical carboné d'au plus 20 carbones, de préférence d'au plus 15 carbones.

Les composés selon la présente invention sont particulièrement intéressants quand leur(s) $R_f$ comporte(nt) plus d'un atome de carbone et surtout plus de trois.

Il est souhaitable que parmi M et X, il y ait au moins un chalcogène plus lourd que l'oxygène.
les composés présentant au moins un atome de sélénium pour M, X, Y, Z, sont particulièrement originaux, les composés où M est chalcogène et où n est égal à 1 présentent la caractéristique de ne donner qu'une perfluoroalcolyation, ce qui est un avantage lorsque c'est cette réaction qui est recherchée. Enfin les dérivés où R correspond à la formule $R_f$-$M(X)(Z)_n$- sont particulièrement intéressant.

Un autre but de la présente invention est de fournir un procédé utile pour greffer des groupements perhalogénés, avantageusement perfluorés, du type $R_f$ précédent. Cette greffe peut être une autogreffe, c'est-à-dire une greffe sur un substrat constitué par un composé ou un produit de décomposition d'un composé de formule (I):

$R_f$-M(X)(Z)$_n$-Y-R ;

Ce but et d'autres qui apparaîtront par la suite sont atteints au moyen d'un procédé comportant au moins une étape dans laquelle on met ledit composé présentant au moins une fonction nucléophile en contact avec un réactif décrit ciavant.

Le procédé selon l'invention va être décrit plus avant en utilisant souvent comme paradigme les thioloesters. Ces thioloesters sont des composés perfluorés qui présentent la formule suivante:

$$R_f - CO - S\text{-}R \qquad \textbf{(III)}$$

dans laquelle :

$R_f$ est défini ci-dessus et représente avantageusement le radical :

$$- C_nF_{(2n+1)}$$

où n est au moins égal à 1, souvent égal à 1,

- et R est un radical aliphatique ou alicyclique, de préférence alcoyle y compris alcényle, alcynyle, aryle, aralcoyle, aralcényle ou aralcynyle, linéaire ou ramifié, substitué ou non.

Il est pratique que les produits de départ, selon l'invention, puissent être constitués par des composés de type $CF_3COSR$, car ce sont des composés très facilement accessibles à partir d'anhydride trifluoroacétique et de thiol RSH.

En outre, le procédé selon l'invention n'implique pas la manipulation, pas plus qu'il n'engendre, de produits dangereux et toxiques.

Ce thioloester perhalogéné, de préférence perfluoré, peut être assimilé à un fournisseur du radical $R_f$*, souvent correspondant à $[C_nF_{(2n+1)}]$* Pour l'obtention des composés visés.

Le procédé selon l'invention peut notamment s'envisager selon deux principales voies :

- la première d'entre elles peut se résumer à une conversion radicalaire directe d'un composé de formule (I) et notamment le thioloester (III) en un perfluoroalcoylthioéther (ou composé perfluoré -S-alcoylé) ($C_nF_{(2n+1)}$ - S-R), par décarbonylation (ou desulfonylation ou équivalents),
- la seconde consiste essentiellement à faire réagir, par voie radicalaire, un composé de formule (I) et notamment le thioloester (III) avec au moins un substrat, de telle sorte qu'au moins un radical $C_nF_{(2n+1)}$ et/ou $C_nF_{(2n+1)}CO$ vienne se greffer sur ledit substrat.

Suivant une modalité avantageuse de l'invention, lesdits mécanismes font intervenir un initiateur, qui peut être de nature chimique (e.g. : peroxyde de benzoyle, peroxyde de tertiobutyle, stannanes, tels que $Bu_3SnSnBu_3$, ou azobisisobutyronitrile = AIBN) et/ou physique (voie thermique et/ou irradiation).

Pour les deux voies du procédé selon l'invention, un mode de mise en oeuvre préféré consiste en une thermolyse du composé (I) entre, de préférence, 350et 650° C (et plus préférentiellement 400 à 600° C) dans, par exemple, un tube de fer, de quartz ou de verre (éventuellement rempli de verre pilé).

Pour les deux voies du procédé selon l'invention, un autre mode préféré de mise en oeuvre consiste en une photolyse dans un milieu réactionnel contenant éventuellement un solvant organique, à une longueur d'onde et à une température réactionnelle $T_R$ ; est, avantageusement, compris entre 200 et 800 nm et, de préférence, entre 210 et 600 nm.

Pour éviter que l'énergie de rayonnement hv initiant la réaction radicalaire, ne soit absorbée par le solvant au détriment du thioloester (I), il importe de choisir le solvant de telle sorte qu'il possède une transparence supérieure ou égale à 50 %, de préférence à 70 % et, plus préférentiellement encore, à 90 %, à la longueur d'onde de travail.

Aussi, conformément à l'invention le solvant est, de préférence, sélectionné parmi les composés suivants : acétonitrile ; éthanol ; butanol ; dichlorométhane ; cyclohexane ; cyclopentane ; dichloro1,2éthane ; diméthyl-2,2butane ; n-heptane ; n-hexane ; méthanol ; méthyl-2butane ; iso-octane ; n-pentane ; propanol-2 ; trichloro-1,2,2trifluoroéthane; trifluoro-2,2,2éthanol ou un mélange de ceux-ci.

Il va de soi que l'invention n'est pas limitée à un mode d'activation par irradiation et notamment par irradiation U.V. Tout autre type d'initiation radicalaire est, en effet, susceptible d'être envisagé : chaleur, auxiliaire radicalaire (cf. Supra).

Suivant une caractéristique intéressante de l'invention, le solvant est choisi de sorte que sa température d'ébullition à pression donnée, soit au moins égale à, de préférence sensiblement égale à la température réactionnelle $T_R$.

Pour obtenir des composés perhalogéno en particulier perfluoroalcoylés et/ou S-alcoylés, il est préférable de prévoir une température réactionnelle TR supérieure ou égale à 20° C et, de façon plus préférée encore, supérieure ou égale à 35° C. En pratique, $T_R$ peut ainsi être de l'ordre de 40° C par exemple.

En revanche lorsque l'on vise des composés perhalogénoacylés, en particulier perfluoroacylés, $T_R$ est idéalement inférieure ou égale à 30° C, de préférence à 25° C et, plus préférentiellement encore, est de l'ordre de 20° C.

Dans le cadre de la deuxième voie de mise en oeuvre du procédé de l'invention, le substrat peut être un disulfure de formule suivante :

R' - S - S - R" **(IV)**

Les radicaux R', R" du composé (IV) et le radical Rdu composé (I) peuvent être identiques ou différents entre eux. Ils sont des préférence identiques et répondent à la définition de R donnée ci-dessus.

A titre d'exemples de radicaux R, R', R" idoines on peut citer :-$CH_2CH_2COOCH_2CH_3$ ; phényle ; chlorophényle ; tertiobutyle ; n-butyle ; cyclohexyle ; benzyle ; méthyle ; isopropyle ; n-propyle ; éthyle ; octyle.

La préparation de thioéthers CnF(2n + 1) SR (V) à l'aide d'un substrat disulfure (IV) bénéficie d'un meilleur rendement que la transformation directe du thioloester (I) en thioéther (V) selon la première voie de mise en oeuvre du procédé selon l'invention.

Selon une variante de sa deuxième voie de mise en oeuvre, le procédé selon l'invention consiste à faire réagir le un composé de formule (I) et notamment le thioloester de formule (III) avec un substrat insaturé au moins partiellement organique, de préférence sous irradiation à une longueur d'onde comprise entre 200 et 800 nm, à une température réactionnelle $T_R$ et dans un milieu organique tel que défini ci-dessus.

On aboutit à un produit perhalogéno, de préférence perfluoro, alcoylé pour $T_R$ 40-50° C et à un produit perhalogéno, de préférence perfluoro, acylé pour $T_R$ 20-30° C.

Les substrats oléfiniques envisageables pour cette perhalogénoalcoylation ou acylation et, en particulier, pour cette trifluorométhylation ($CF_3$-) ou trifluoroacétylation ($CF_3$-CO-) à l'aide de thioloesters (I) de l'acide trifluoroacétique ($CF_3COSR$) sont, notamment, les alcènes, les cycloalcènes, les (cyclo) alcynes, les aromatiques ou les mélanges de ceux-ci.

De préférence, le substrat oléfinique est caractérisé en ce qu'il est formé par au moins l'un des produits appartenant à au moins l'une des classes chimiques suivantes :

- alcènes et alcynes non fonctionnalisés sur l'insaturation,
- éthers et esters d'énols,
- perhalogénocarboxylates d'énols,
- sulfures de vinyle,
- énamines,
- éneoxy, énethio et énéaminostannanes,
- allystannanes,
- éneoxy, énethio et éneaminosilanes,
- allylsilanes.

Concernant la stoechiométrie propre aux formes préférées de mise en oeuvre du procédé selon l'invention (2ème voie), il est à considérer que le rapport molaire entre le composé de formule (I) et le substrat est avantageusement compris entre 0,1 et 10, de préférence entre 0,5 et 2 et, plus préférentiellement encore, entre 0,9 et 1,1.

lorsque l'on fait réagir le réactif selon l'invention sur une double liaison carbone-carbone ; le radical libre résultant peut évoluer de diverse façons et notamment se combiner avec les radicaux soufrés du milieux ou bien arracher un hydrogène à un composé en portant . Si l'on désire favoriser la réaction d'arrachage d'hydrogène (car donnant l'addition du perfluoroalcoyle sur un des carbones et d'un hydrogène sur l'autre) il convient d'introduire dans le milieu réactionnel des produits, qui tel le diphénylméthane, le toluène, les xylènes, ou mêmes les allyles, sont capables de donner un atome d'hydrogène à un radical libre.

Conformément à l'invention, les composés de formule (I) sont des nouveaux agents de perhalogénoalcoylation et/ou -S-alcoylation et/ou acylation radicalaire, l'halogène considéré étant, de préférence, le fluor. Ces thioloesters

(I) ont l'avantage d'être faciles à préparer et à mettre en oeuvre, économiques et écologiquement tolérables.

Il est sans doute superfétatoire de noter que seuls les composés selon la formule où M est carbone donnent des acylations.

Les perhalogéno(fluoro)alcoylthioéthers et les produits perhalogéno(fluoro) alcoylés ou acylés obtenus par le procédé selon l'invention sont notamment exploitables dans le domaine pharmaceutique ou phytosanitaire.

mais une des applications les plus innovantes et les plus intéressants consiste à faire des dérives à la fois silanés et présentant au moins une ramification perfluorée.

ces composés difficiles à préparer présente un grand intérêt. En utilisant la technique selon la présente invention, il est notamment possible de réaliser une telle synthèse soit par perfluoration d'un dérivé présentant une fonction silane et une insaturation en position non vinylique soit en perfluorant un composé présentant deux doubles liaisons puis en silylant la fonction restante

Ainsi selon l'invention Il particulièrement intéressant de soumettre au procédé selon l'inventio des composés de formule V ci après :

$$(R_4)(R_5)C{=}C(R_6)(R_7)$$

avec $R_4$, $R_5$, $R_6$, $R_7$ semblables ou différents choisis parmi l'hydrogène, les radicaux hydrocarbonés et notamment parmi les alcoyles, y compris les cycloalcoyles et les aralcoyles, et parmi les aryles avec la condition que au moins un des $R_4$, $R_5$, $R_6$, $R_7$ représente :

- un radical monovalent portant un atome de silicium éloigné de la valence libre d'au moins de un, avantageusement d'au moins deux, atomes de carbone ;

les radicaux $R_4$, $R_5$ peuvent être reliés à leur radical correspondant (c'est à dire en position cis) $R_6$ ou $R_7$ pour former un ou deux cycles avantageusement non aromatique. Il est toutefois préférable qu'il n y ait qu'un cycle ainsi formé

Les produits ainsi formés sont des formule VI :

$$(R_4)(R_5)CH - C(\mathbf{Rf})(R_6)(R_7) \qquad (VII)$$

Dans laquelle $R_4$, $R_5$, $R_6$,et $R_7$ ont la même valeur que pour la formule (V).

Rf présente avantageusement au moins deux, de préférence au moins quatre carbone. En outre avantageusement p+m est au moins égal à deux, de préférence au moins à quatre.

il est souhaitable qu'un seul des $R_4$, $R_5$, $R_6$,et $R_7$ porte une double liaison non aromatique ou un (ou plusieurs) atome(s) de silicium ;

Comme la réaction de perfluoroalc(o)ylation est très sensible à l'encombrement stérique du substrat, il est préférable que parmi les radicaux $R_4$, $R_5$, $R_6$,et $R_7$, Il y en ait au moins 1, de préférence au moins 2 qui représente des atomes d'hydrogène.

Il est préférable que parmi les radicaux $R_4$, $R_5$, $R_6$,et $R_7$, Il y en ait au plus un qui représentent un radical encombrant. Par radical encombrant on entend dans la présente description un radical dont l'atome porteur de la valence libre porte en outre deux et surtout trois ramifications d'au moins un carbone.

Compte tenu de ce qui précède, il convient de signaler que la réaction est très sélective entre les doubles liaison peu encombrées et celle qui le sont.

L'invention sera mieux comprise, ses avantages et ses variantes de mise en oeuvre ressortiront bien des exemples qui suivent et qui décrivent la préparation de thioéthers et de composés perfluoroalcoylés ou acylés à l'aide de thioloesters (I) d'acide perfluorocarboxyliques, que ladite invention concerne.

## EXEMPLES

### EXEMPLE 1 : SYNTHESE DES THIOLOESTERS DE FORMULE (I)

Pour ce qui est de la synthèse, d'une manière générale les thioloesters ne se distinguent pas des techniques connues de l'homme de métier pour les thioloesters non fluorés.

$R_fCO)_2O$ + RSH  excès  $CH_2Cl_2$, DMAP / Pyridine (1 éq.)  $R_f$ -CO-S-R

On utilise comme exemple paradigmatique de cette réaction les dérivés de l'acide trifluoro acétique.

$CF_3CO)_2O$ + RSH  excès  $CH_2Cl_2$, DMAP / Pyridine (1 éq.)  $CF_3CO$-S-R

DMAP = 4-DiMéthylAmino Pyridine.

**1.1** MODE OPERATOIRE GENERAL:

Dans un tricol de 500 ml, on introduit 150 ml de dichlorométhane (distillé et séché sur tamis moléculaire 4 Angström) sous courant d'azote.

On ajoute ensuite la pyridine (4 ml, 50 mmol), le thiol (50 mmol) et une pointe de spatule de DMAP.

Le milieu réactionnel est refroidi à 0° C par un bain de glace et on additionne goutte à goutte une solution d'anhydride trifluoroacétique (8 ml, 57 mmol) dans 50 ml de $CH_2Cl_2$ anhydre.

L'addition dure environ 15 minutes.

La réaction est suivie par Chromatographie en Phase Vapeur (CPV).

On laisse le milieu réactionnel 1 heure à température ambiante et dans le cas d'un thiol peu nucléophile, on peut porter à ébullition 1 heure supplémentaire.

On coule le milieu réactionnel dans 150 ml d'$H_2O$ et on extrait deux fois avec $CH_2Cl_2$. La phase organique est séchée sur $MgSO_4$, filtrée et concentrée dans un évaporateur rotatif sous vide de la trompe à eau sans chauffer. Le produit brut obtenu est distillé.

**2.2** RESULTATS :

**TABLEAU I**

| - R : | Rendement $CF_3COSR$ isolé par rapport au thiol (%) | $Eb_p$ | | PF |
|---|---|---|---|---|
| | | P = 20 | P = 760 | |
| - $CH_2CH_2CO_2Et$ | 95 | 101-102 | - | - |
| - Ph | 96 | 69-74 | - | - |
| - 4 - Cl - Ph - | 87 | 123-124 | - | - |
| - tertiobutyle | 63 | - | 99 | - |
| - Cyclohexyle | 80 | 94 | - | - |
| $PhCH_2$ | 84 | 115-117 | - | - |
| - Et | Produit du Commerce | - | - | - |
| - $(CH_2)_7CH_3$ | 97,5 | 120 | - | - |
| $CH_2CO_2Et$ | 81 | 90 | - | - |
| (*) $-CH_2CH(NH_3Cl)CO_2R^t$ (L) $R^t$ = Me<br>$R^t$ = Et | 94<br>94 | -<br>- | -<br>- | 78<br>73 |

ABREVIATIONS :

$Eb_p$ = température d'ébullition en °C à une pression P en mm Hg,

PF = point de fusion en °C à 760 mm Hg,

Et = éthyle, Ph = phényle, 4 - Cl - Ph = p - chlorophényle.

P = pression en mm de mercure

* = Dans le cas des esters de la L-cystéine, le mode opératoire est différent :

$(CF_3CO)_2O$ + $HSCH_2CH(NH_3Cl)CO_2R^t$ — acétate d'éthyle, 25° C — $CF_3COSCH_2CH(NH_3Cl)CO_2R^t$

4 éq 1 éq.

**EXEMPLE 2 : PREPARATION DE DIFFERENTS THIOETHERS $CF_3$-S-R (III).**

$$R_fCOSR + R\text{-}S\text{-}S\text{-}R \xrightarrow[h\nu,\ 40°C]{CH_2Cl_2,\ (20\ ml)} R_f\text{-}S\text{-}R_+\ RSSR$$

On utilise comme exemple paradigmatique de cette réaction les dérivés de l'acide trifluoroacétique.

$$CF_3COSR + R\text{-}S\text{-}S\text{-}R \xrightarrow[h\nu,\ 40°C]{CH_2Cl_2,\ (20\ ml)} CF_3\text{-}S\text{-}R_+\ RSSR$$

**2.1** PROTOCOLE GENERAL :

Dans une cuve en quartz on introduit 20 ml (sauf pour l'essai 1 : 50 ml) de dichlorométhane distillé et anhydre (séché sur tamis moléculaire 4 Angstrom) que l'on désoxygène à l'azote pendant 15 minutes.

On introduit ensuite le thioloester et le disulfure et on irradie avec une lampe UV à vapeur de mercure haute pression, de type HPK 125 (puissance = 125 W) et de marque déposée PHILIPS® (sauf pour l'essai 1 : NK 12 basse pression HANOVIA®).

La réaction est suivie par CPV.

On peut faire un dosage par RMN $^{19}F$ (200 MHz) du produit brut en fin d'irradiation, en présence de (trifluorométhoxy)benzène comme étalon interne. Le milieu réactionnel est ensuite concentré sans chauffer sous vide de la trompe à eau. Les produits sont séparés par chromatographie sur colonne de silice.

**2.2** RESULTATS:

Le tableau II, annexé aux pages 17 et 18, présente les conditions réactionnelles et les données recueillies pour différents substituants R.

## TABLEAU II

| R | Numéros des essais | $CF_3COSR$ (I) (mmol) | RSSR (II) (mmol) | Temps | $CF_3SR$(III) (mmol) | $CF_3COSR$(I) non réagi(mmol) | RSSR(II) récupéré (mmol) | RR (%) | TT[1] (%) | TT[2] (%) |
|---|---|---|---|---|---|---|---|---|---|---|
| $CH_2CH_2$-CO-OEt | 1 | 2,5 | 2,3 | 4 h | 0,90 | 0 | 2,3 | 36 | 54 | 0 |
| | 2 | 1,88 (2 lots) | 0 | 2 x 1 h 15 | 0,60 (0,58) | 0,13 | 0,245 | 32 | 93 | - |
| | 3 | 1 | 0,95 | 1 h 45 | 0,64 (0,63) | (0,21) | 0,75 | 64 | 79 | 21 |
| | 4 | 0,97 | 0,36 | 1 h 15 | 0,48 | 0 | 0,36 | 49,5 | [90 ] | 0 |
| | 5 | 0,96 | 1,95 | 1 h 15 | 0,64 | 0 | 1,80 | 67 | - 80 | 8 |
| | 6 | 0,95 | 0,18 | 1 h 20 | 0,42 | 0 | 0,23 | 44 | 97 | - |
| 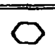 | 7 | 2,01 (2 lots) | 0 | 2 x 1 h 15 | 0,35 (0,51) | [0,19] | 0,39 | 19,5 (25,5) | 90,5 | - |
| | 8 | 0,94 | 1,04 | 1 h 30 | 0,20 (0,28) | [0,16] | 1,05 | (30 ) | 72 | 0 |
| $C[(CH_3)]_3$ | 9 | 1,1 | 0 | 1 h | (0,22) | (0,042) | non dosé | 20 | 96 | - |
| $(CH_2)_7CH_3$ - | 10 | 2 x 0,95 | 0 | 2 x 1 h 15 | 0,72 (0,59) | (0,02) | 0,31 | 38 (31) | 99 | - |
| | 11 | 0,95 | 0,93 | 1 h 45 | 0,63 (0,68) | (0,08) | 0,93 | 66 (72 ) | 92 | 0 |
| -Φ | 12 | 1,1 | 0 | 1 h 30 | (0,16) | (0,19) | 0,42 | 14,5 | 83 | - |
| | 13 | 1,1 | 0,88 | 3 h 00 | 0,17 (0,29) | (0,25) | 0,50 | 26 | 77 | 43 |
| -Φ Cl | 14 | 1,15 + 1,2 | 0 | 2 x 1 h 30 | 0,41 (0,42) | (0,35) | 0,53 | 17,5 | 85 | - |
| | 15 | 1,1 | 1,07 | 3 h 30 | 0,29 (0,31) | (0,39) | 1,29 | 27 | 75 | - |
| $-CH_2$-Φ | 16 | 1,09 + 1,1 | 0 | 1 h 30 | 0,16 (0,20) | (0,18) | [0,12] | 7,5 (9) | 92 | - |
| - $CH_2CO_2Et$ | 17 | 2,08 (2 lots) | 0 | 2 x 1 h 20 | 0,34 (0,58) | (0,106) | 0,17 | 16 (28) | (95) | - |
| | 18 | 0,97 | 1 | 2 h 05 | (0,445) | (0,12) | 0,84 | (46) | (87) | 16 |
| L $CH_2CH(CO_2Me)$ $(NHCOCF_3)$ | 19 | 0,86 | 0 | 2 h 15 | 0,27 (0,175) | 0 | 0,17 | 31 | 100 | - |
| | 20 | 1,00 | 1 | 1 h 50 | 0,60 | 0 | 1,09 | 60 | 100 | - |

**TABLEAU III**

| R | Numéros des essais | $CF_3COSR$ (I) (mmol) | RSSR (II) (mmol) | Temps | $CF_3SR$ (III) (mmol) | $CF_3COSR$ (I) non réagi (mmol) | RSSR (II) récupéré (mmol) | RR (%) | TT[1] (%) | TT[2] (%) |
|---|---|---|---|---|---|---|---|---|---|---|
| | 20 | 1,00 | 1 | 1 h 50 | 0,60 | 0 | 1,09 | 60 | 100 | |

# TABLEAU IV

| Numéros des essais | x (mmol) (I) | y (mmol) (V) | v (ml) | t (h) | $CF_3COSR$ (I) | | (VI) | | (VII) | | (III) | |
|---|---|---|---|---|---|---|---|---|---|---|---|---|
| | | | | | Nbre de moles n'ayant pas réagi | TT (%) | Nbre mmol | RR (%) | Nbre mmol | RR (%) | Nbre mmol | RR (%) |
| 22 | 0,98 | 5 | 20 | 1 h 45 | (0,25) [0,22] | 76 | (0,37) | 38 | (0,075) | 7,5 | (0,11) | 11 |
| 23 | 1 | 4,95 | 10 | 6 h 30 | (0,225) | 77,5 | (0,38) | 38 | (0,11) | 11 | (0,10) | 10 |
| 24 | 2 | 9,9 | 5 | 10 h 20 | (1,1) | 45 | (0,37) | 18,5 | (0,21) | 10,5 | (0,15) | 7,5 |
| 25 | 1,2 | 1 | 20 | 1 h 45 | [0,38] (0,41) | 67 | (0,35) | 29 * | (0,015) | 1 * | (0,13) | 11 |
| 26 | 0,96 | 2,5 | 20 | 1 h 45 | [0,25] (0,28) | 72 | (0,27) | 28 | (0,04) | 4 | (0,10) | 10,5 |
| 27 | 1 | 0,4 | 20 | 0 h 45 | [0,40] (0,53) | 47 | 0,22 | 55 * | 0 | 0 | (0,11) | 11 |
| 28 | 0,95 | 4,95 | 20 | 1 h 45 | - | 73 | - | 24 | - | 18 | - | 12,5 |
| 29 | 1,4 | 1 | 10 | 2 h 00 | 0,72 | 49 | 0,38 | 38 * | - | - | 0,17 | 12 |
| 30 | 1 | 5 | 10 | 6 h 35 | - | (75) | - | (28) | - | (10) | - | (8,5) |
| 31 | 1 | 5 | 10 | 4 h 00 | - | (87) | - | (34) | - | (13) | - | (9,5) |
| 32 | 1 | 5 | 20 ** | 1 h 30 | - | (63) | - | (11) | - | (14) | - | (3,3) |

* = vs cyclohexène

** solvant = chlorobenzène

- RR = rendement en composé obtenu par rapport au réactif (I) ou substrat (II) introduit :
- $TT^1$ = taux de transformation du réactif (I) égal à :
- $TT^2$ = taux de transformation du réactif (II) égal à :
- RT = rendement en composé obtenu par rapport au réactif (I) ou substrat (II) consommé :

Dans ce tableau II, les résultats entre parenthèses sont obtenus par dosage RMN $^{19}F$ avec étalon interne.

**EXEMPLE 3: PREPARATION DE DIFFERENTS THIOETHERS $CF_3$ - S - $R^3$.**

2.1 PROTOCOLE GENERAL:

$$\underset{(I)}{CF_3COSR} + \underset{(II)}{R\text{-}S\text{-}S\text{-}R} \xrightarrow[h\nu\ /\ 40°C\ /\ 1h30]{CH_2Cl_2\ (20\ ml)} \underset{(III)}{CF_3\text{-}S\text{-}R} + \underset{(IV)}{RSSR}$$

avec :

- R = t-Bu,
- $R^1 = R^2 = CH_2CH_2CO_2$ Et,
- $R^3 = R^1 = R^2 = CH_2CH_2\ CO_2$ Et.

La méthodologie mise en oeuvre est identique à celle de l'exemple 2.
Le tableau III, présente les résultats obtenus en fonction des conditions réactionnelles.

**EXEMPLE 4: PERFLUOROALCOYLATION ET PERFLUOROACYLATION DE CYCLOHEXENE A L'AIDE D'UN REACTIF DE FORMULE (I) : $CF_3$COSR.**

$CF_3$-CO-SR + cyclohexène $\xrightarrow{CH2Cl2\ °C\ h\nu}$ cyclohexyltrifluorométhane (VI) + la trifluorométhyl cyclohexylcétone (VII) et $C_3$F-S-R (III)

L'irradiation UV s'effectue à l'aide de la même lampe que pour les exemples 2 et 3.
Dans une première série d'essais (essais 22 à 28), le substituant R de (I) =$-CH_2CH_2CO_2$Et avec une température réactionnelle de 40° C pour les essais 22 à 27 et de 20° C pour l'essai 28.
La deuxième série comprend les essais 29 et 30 réalisés à $T_R$ = 40°C pour R = - $CH_2CH_3$.
La troisième série d'essais (31 et 32) correspond à un substituant R = Ph, avec $T_R$ = 40° C pour l'essai 31 et $T_R$ = 20° C pour l'essai 32.
Le tableau IV, annexé à la page 19, montre les conditions d'essais ainsi que les résultats.
Dans ce tableau IV, les résultats signalés par les parenthèses "()" ont été obtenus par dosage par RMN $^{19}F$ (avec étalon interne) et ceux signalés par les crochets "[]"par dosage CPV (avec étalon interne).

**EXEMPLE 5: PERFLUOROALCOYLATION D'OLEFINES.**

**5.1** TETRADECENE-7 (VIII) :

Le mode et les conditions opératoires sont donnés ci-dessous. Dans un premier temps, il s'agit de la perfluoroalcoylation (cf. Exemple 4) :

$$CF_{3}\text{-}CO\text{-}SR + C_{6}H_{13}\text{-}CH{=}CH\text{-}CH_{2}\text{-}C_{5}H_{11}\ (VIII) \rightarrow C_{6}H_{13}\text{-}C(CF_{3})H\text{-}CH_{2}\text{-}CH_{2}\text{-}C_{5}H_{11}\ (IX) +$$

$$C_{6}H_{13}\text{-}C(CF_{3})H\text{-}CH{=}CH\text{-}C_{5}H_{1}(X)+$$

$$CF_{3}\text{-}SR\ (III)\ (\text{avec } R = \text{éthyle})$$

conditions opératoires :

- durée 3 heures
- température : à 40°C.
- activation par irradiation dans le chlorure de méthylène dans les conditions ci-dessus.

Les résultats obtenus par dosage RMN $^{19}F$ sont donnés dans le tableau V ci-dessous.

TABLEAU V

| N° ESSAI | $CF_3COSEt$ (mmol) (I) | Oléfine (mmol) (VIII) | $CF_3COSEt$ (I) restant | | Oléfine restant e (VIII) | | (IX) | | (X) | | (III) | |
|---|---|---|---|---|---|---|---|---|---|---|---|---|
| | | | mmol | TT (%) | mmol | TT (%) | nb mmol | RR (%) | nb mmol | RR (%) | nb mmol | RR (%) |
| 33 | 1,8 | 1,1 | (0,45) | 75 | 0 | 100 | (0,43) | 39 | (0,04) | 3,5 | (0.4) | 22 |

Dans un second temps on met en oeuvre une réduction (hydrogénation) pour permettre la transformation de (X) en (IX). conditions utilisées : $H_2$/Pd/C (10 %)/2 bar(= 2 $10^5$ pascals) :

$RR_{global}$ = 41 % par rapport à l'oléfine (VIII).

**5.2** DODECENE-1 (XI) :

5.2.1 Perfluoroalcoylation (cf. Exemple 4) :

conditions opératoires :

- durée 3 heures et demie ;
- température : à 40°C.
- activation par irradiation dans le chlorure de méthylène dans les conditions ci-dessus.

$CF_3$ - COSEt+ $CH_2$ = CH-$(CH_2)_9$-$CH_3$ $\xrightarrow{CH2Cl2.h\nu}$ > * $CF_3(CH_2)_{11}CH_3$ **(XII)**

$CF_3CH_2$ - $CH(CH_2)_9CH_3$ **(XIII)**

* $CF_3SEt$ **(III)**

$CF_3CH$ = CH - $(CH_2)_9CH_3$

* $CF_3CH_2$ - CH = CH - $(CH_2)_8CH_3$

RESULTATS :

TABLEAU VI

| N° ESSAI | $CF_3COSEt$ (I) (mmol) | Oléfine (XI) (mmol) | $CF_3C$ OSEt restan t | | Oléfine restante | | (XII) | | (XIII) | | (III) | |
|---|---|---|---|---|---|---|---|---|---|---|---|---|
| | | | mmol | TT (%) | mmol | TT (%) | nb mmol (%) | RR | nb mmol | RR (%) | nb mmol | RR (%) |
| 34 | 1,56 | 0.96 | 0,62 | 60 | 0 | 100 | (0,34) | 35,5 | (0,07) | 7,5 | (0,325) | 21 |

COMMENTAIRES:

On isole 110 mg d'un mélange contenant, en proportion par RMN $^{19}F$:

- 85,5 % de $CF_3(CH_2)_{11}CH_3$,
- 14,5 % de $CF_3CH = CH - (CH_2)_9CH_3$ et $CF_3CH_2 - CH = CH(CH_2)_8CH_3$.

5.2.2 Réduction :

Dans un second temps on met en oeuvre une réduction pour permettre la l'hydrogénation des oléfines [$H_2$/Pd/C (10 %)/2 bar(=) 2 $10^5$ pascals dans acide acétique]:

$RR_{global}$ = 46 % par rapport à l'oléfine (XI).

**EXEMPLE 6** : **SYNTHESE D'ALCOOLS PERFLUOROALCOYLES PAR PERFLUOROALCOYLATION D'ACETATES D'ENOLS.**

On irradie un acétate d'énol en présence de $CF_3COSEt$ (I), dans les conditions de l'exemple 4. Après évaporation du chlorure de méthylène, le mélange résultant de composés trifluorométhylés est repris dans l'éther éthylique et traité à 20° C avec de l'hydrure de lithium et d'aluminium :

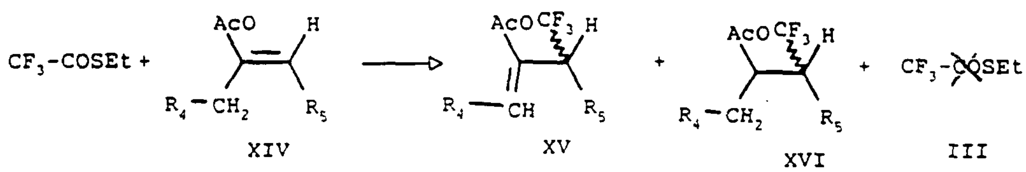
XIV XV XVI III

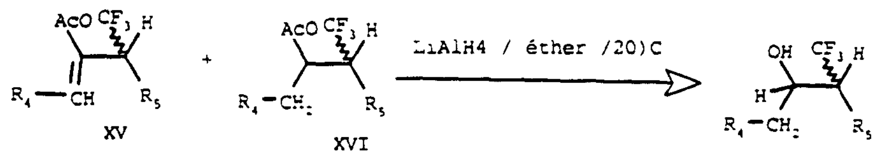

XV XVI

deux diastéréoisomères XVII

RESULTATS :

TABLEAU VII

| $R^4$ | $R^5$ | x mmol | y mmol | t h | (I) récupé ré | | Oléfine récupé rée (XIV) | | (XVI) | | (XV) | | (III) | | z mmol | (XVII) | |
|---|---|---|---|---|---|---|---|---|---|---|---|---|---|---|---|---|---|
| | | | | | mmol | $TT^1$ % | mmol | $TT^2$ % | mmol | RR % | mmol | RR % | mmol | RR % | | mmol | RR% |
| $R^4 - R^5 = (CH_2)_3$ | | 1,4 | 0,99 | 2 h05 | (0.34) | (76) | 0 | 100 | (0,26) | (26) | (0,03) | (3) | (0.325) | (23) | 0,9 | 0,3 | 30 |
| n-$C_4H_9$ | n-$C_4H_9$ | 2x1,4 | 0,99 +0.90 | 2x 2h05 | 0.40 | 86 | 0,55 | 71 | - | - | - | - | 0,73 | 26 | 7,5 | 0,4 | 21 |
| | | 3x0,7 | 3x 0,455 | 3x3h | - | - | 0 | 100 | - | - | - | - | - | - | 4 | 0,42 | 31 |

**EXEMPLE 7 : PREPARATION DE $CF_3$ - S - Ph PAR THERMOLYSE DE $CF_3$CO-S-Ph.**

Un tube de Pyrex en spirale de 2 m de longueur et 4 mm de diamètre est porté pendant 4 heures à 600° C sous atmosphère d'argon dans un four cylindrique. Il est ensuite placé, successivement, aux différentes températures indiquées dans le tableau VIII infra. Dans chaque expérience, 0,5 g de trifluorothioacétate de S-phényle (I) est introduit dans un injecteur relié à ce four. Ce composé est porté à ébullition et traverse le tube sous pression autogène. Les produits sont récupérés dans un ballon refroidi par de la glace. La déperdition en poids est faible. Le mélange de composés fluorés contient du trifluorométhylphénylsulfure (III) et du produit de départ (I). Le tableau VII suivant indique les proportions en fonction de la température.

| RESULTATS :TABLEAU VIII | | |
|---|---|---|
| T (° C) | (I) (%) | ((III) (%) |
| 400 | 98 | 2 |
| 450 | 95 | 5 |
| 500 | 80 | 20 |
| 550 | 75 | 25 |

**EXEMPLE 8** :*Synthèse de dérivés de formule* $R_f$-M(X)(Z)$_n$-Y-R *lorsque M est chalcogène* (M = S, Se) *en utilisant* $CF_3SO_2YR$ (Y = S, Se) *comme paradigme de la famille.*

*Synthèse de* $CF_3SO_2YR$ (Y = S, Se)

- *1ère méthode*

$$2\ R_fMO_2^- + R\text{-}Y\text{-}Y\text{-}R + Br_2 \rightarrow .2\ R_f\text{-}MO_2\text{ - }Y\text{-}R + 2Br$$

Dans un ballon contenant 2 équivalents de triflinate en suspension dans $CH_2Cl_2$ on ajoute 1 équivalent de disulfure (diséléniure) puis on coule 1 équivalent de Brome en solution 1M dans le $CH_2Cl_2$.
8h00 après (suivi CPV), le mélange réactionnel est filtré, le solvant évaporé et le composé est purifié par distillation ou recristallisation dans l'éther de pétrole.

- *2ème méthode*

$$R_f\text{-M(O)(Z)- } + R\text{-}Y\text{-}Cl \rightarrow R_f\text{-}MO\text{(Z) - } Y\text{-}R + Cl^-$$

Dans un ballon contenant 1 équivalent d-e triflinate en suspension dans $CH_2Cl_2$, on coule 1 équivalent de chlorure de sulfényle (sélénényle) en solution dans le $CH_2Cl_2$. La réaction est relativement rapide.
Le mélange réactionnel est filtré et le solvant évaporé ; le composé est propre.

| Composé | Méthode de synthèse | Rendement | RMN$^{19}$F ($CDCl_3$ par rapport à $CFCl_3$) | Pt de fusion |
|---|---|---|---|---|
| $CF_3SO_2SCH_2CO_2Et$ | 1 | 88 % | - 78,95 | - |
| $CF_3SO_2S\ CO_2Me$ | 2 | 96 % (dosage RMN $^{19}$F | - 75,91 | - |
| $CF_3SO_2S(CH_{2)7}CH_3$ | 1 | 88 % | - 78,96 | - |
| $CF_3SO_2S$ -tBu | 1 | 0 % | - | - |
| $CF_3SO_2S$ -cyclohexyle | 1 | 65 % | - 79,25 | - |

(suite)

| Composé | Méthode de synthèse | Rendement | RMN19F ($CDCl_3$ par rapport à $CFCl_3$) | Pt de fusion |
|---|---|---|---|---|
| $CF_3SO_2S$-Φ | 12 | 50%-67% 84 % | - 75,8 | 38 - 39°C |
| $CF_3SO_2S$-Φ-$NO_2$ | 2 | 93 % | - 75,32 | 76 - 77°C |
| $CF_3SO_2S$-Φ-Cl | 1 | 50 % | - 75,46 | - |
| $CF_3SO_2S$-$CH_2$-Φ | 1 | 90 % | - 78,64 | - |
| $CF_3SO_2S$-CCl3 | 2 | 95 % | - 75,2 | - |
| $CF_3SO_2Se$-Φ | 2 | 95 % produit instable | - 77,6 | non determinable car solide qui se dégrade thermiquement |

*Synthèse de $CF_3$ SR par photolyse*

Dans une cuve en quartz de 20 ml, 1 mmole de $CF_3SO_2SR$ est introduite dans 20 ml de $CH_2cl_2$ anhydre. 1 mmole de disulfure correspondant est ensuite introduite. Le mélange réactionnel est dégazé pendant 10 minutes à l'azote sec puis est irradié 40 minutes à 40°c (reflux du solvant) avec une lampe UV HPK 125 Phillips (125 W, vapeur de mercure, haute pression).

Le produit attendu est ensuite purifié par chromotagraphie éclair. Le disulfure de départ est récupéré dans la quasi-totalité.

*$CF_3$ Se R*

- Dans un ballon contenant 1 équivalent de triflinate en suspension dans $CH_2cl_2$ et 2 équivalents de diséléniure, 1 équivalent de chlorure de sélénényle correspondant est coulé.
  A la fin de l'addition, le mélange réactionnel est chauffé à 40°c pendant 24h00.
  Le produit attendu est purifié par chromatographie éclair. Le diséléniure de départ est récupéré dans sa quasi-totalité.
- Ce composé peut également être obtenu sans utiliser de chlorure de sélénényle. Suivant la même procédure que précédemment mais avec 3 équivalents de diséléniure au départ, on coule alors 1 équivalent de $Br_2$ en solution 1M dans $CH_2cl_2$ à la place du chlorure de sélénényle.

| Composé | Rendement | | RMN19F (CDCl3 par rapport à $CFCl_3$) |
|---|---|---|---|
| | Dosage RMN19F | Isolé | |
| $CF_3$ S-Φ | 25 % | 20 % | - 43,37 |
| $CF_3$ S $(CH_2)$7 $CH_3$ | 80 % | 74 % | - 41,58 |
| $CF_3S$ $CH_2$ $CH_2$ $CO_2$ Et | 78 % | 65 % | - 41,87 |
| $CF_3$ Se Méthode avec RSecl | 90 % | 74 % | |
| $CF_3Se$ Méthode avec Br2 | 80 % | 70 % | - 36,6 |

**EXEMPLE 9 :*Greffage photochimique sur des oléfines***

Dans une cuve en quartz de 20 ml, 1 mmole de $CF_3SO_2SR$ est introduite dans 20 ml de $CH_2Cl_2$ anhydre. 1 mmole d'alcène est introduite. Le mélange réactionnel est dégazé pendant 10 minutes à l'azote sec puis est irradié 1h15' avec une lampe Phillips HPK125 (125 W, vapeur de mercure, haute pression).

Les produits sont dosés par RMN19F et identifiés par couplage chromatographie gazeuse/spectrométrie de masse.

| ALCENE ALCENE | Nombre d'équivalents de $CF_3SO_2S\Phi$ | Rendement (par Dosage) | Produit |
|---|---|---|---|
| $CH_2$= CH-$(CH_2)_8$-$CH_3$ | 1 | 36 %<br>12 %<br><br><br>11 % | $CF_3$- $(CH_2)_{10}$-$CH_3$<br>$CF_3$CH= CH-$(CH_2)_8$-$CH_3$ OU $CF_3$-$CH_2$-CH= CH-$(CH_2)_8$-H<br>$CF_3$-CH-CH(S$\Phi$)-$(CH_2)_9$-H |
| CH2= CH-$(CH_2)_8$-$CH_3$ | 2 | 33 %<br>49 % | $CF_3$- $(CH_2)_{10}$-$CH_3$<br>$CF_3$-CH-CH(S$\Phi$)-$(CH_2)_9$-H |
| $CH_2$= CH$(CH_2)_8$-$CH_3$ | 3 | 30 %<br>46 % | $CF_3$- $(CH_2)_{10}$-$CH_3$<br>$CF_3$-CH-CH(S$\Phi$)-$(CH_2)_9$-H |
| $CH_2$= CH-C$(CH_3)_2$-$CH_2$-COOMe | 2 | 45 %<br><br>45 % | $CF_3$-$CH_2$- $CH_2$-C$(CH_3)_2$-$CH_2$-COOMe<br>$CF_3$-CH-CH(S$\Phi$)-C$(CH_3)_2$-$CH_2$-COOMe |
| CYCLOHEXENE | 1 | 26 %<br>13 %/traces | CYCLOHEXYLE -$CF_3$<br>trifluoromethyl-1 thiophényle-2 cyclohexane (2 diastéréoisomères) |
| CYCLOHEXENE | 2 | 25 %<br>30 %/10 % | CYCLOHEXYLE -$CF_3$<br>trifluoromethyl-1 thiophényle-2 cyclohexane (2 diastéréoisomères) |

**EXEMPLE 10 :Synthèse de pentadécafluorothioctanoate d'octyle $C_7F_{15}COS(CH_2)7CH_3$ 4**

Dans un ballon tricol de 50 ml muni d'un thermomètre, d'une agitation magnétique et surmonté d'un réfrigérant ascendant, on introduit 12 ml de dichlorométhane anhydre, 0,86 ml (5 mmol) de n-octanethiol et 0,90 g (7,5 mmol) de diméthylaminopyridine. Le milieu réactionnel est refroidi à 0°C à l'aide d'un bain de glace. Une solution de chlorure de pentadécafluorothioctanoyle (1,6 ml, 6,5 mmol) dans 10 ml de dichlorométhane anhydre est alors ajoutée goutte à goutte en 20 minutes.

En fin d'addition, on laisse remonter la température du milieu réactionnel et on le maintient à température ambiante pendant 2h30, puis on le chauffe à reflux pendant 1 h. On ajoute, ensuite, 0,135 ml (2,5 mmol) d'acide trifluoacétique. On purifie le produit par chromatographie éclair sur silice (10 g) avec le dichlorométhane comme éluant. Les phases de nature identiques (CPV) sont rassemblées et séchées sur sulfate de magnésium. Le solvant est éliminé par évaporation sous le vide de la trompe à eau à eau à température ambiante. On obtient 2,29 g d'un liquide incolore avec une odeur nauséabonde (rdt = 90 %).

RMN'H (200,13 MHz, CDCI3)
S (ppm) = 0,9 (m, 3H, $C\underline{H}_3$) ; 1,3 (m, 10 H) ; 1,6 (m, 2H, $SCH_2$ $C\underline{H}_2$) ; 3,1 (t,3 = 7,3 H2, 2H, $S\underline{C}H_2$).

RMN$^{19}$F : (188,35 $MH_2$, $CDCl_3$) $CF_3$ - $CF_2^f$ - $CF_2^e$ - $CF_2^d$ - $CF_2^c$ - $CF_2^b$-$CF_2^a$$COSC_8M_{17}$
S(ppm) = - 81,52 (t, $h_J$ = 9,5 $H_2$, 3F, $C\underline{F}_3$) ; - 116,29 (t, hJ = 13,2 $H_2$, 2F, $C\underline{F}_2^a$)
- 121,81 (pic large, 2F, $C\underline{F}_2^b$) ; - 122.42 (pic large, 4F,$C\underline{F}_2^c$, $C\underline{F}_2^d$) ;
- 123,20 (pic large, 2F, $C\underline{F}_2^a$) ; - 126,69 (pic large, 2F, $C\underline{F}_2^f$).

RMN$^{13}$C (50,32 MHz, CDCl3)
S(ppm) = 13,78 (CH3) ; 22,63 à 31,81 (7 CH2) ; 108,96 à 120,79 (massifs non résolus, CFn). ; - 186,52 (CO).

**EXEMPLE 11 :Synthèse de pentadécafluorothiooctanoate de méthyle $C_7F_{15}COSCH_3$ 5**

Dans un ballon tricol de 50 ml muni d'un septum, d'un thermomètre, d'une agitation magnétique et surmonté d'un réfrigérant ascendant, on introduit le chlorure de méthylène (5 ml) puis $CH_3SNa$ (0,14 g, 2 mmol). On refroidit le milieu réactionnel à l'aide d'un bain de glace, on le maintient sous atmosphère d'azote et on coule goutte à goutte, en 10 minutes, une solution de $C_7F_{15}COCl$ (0,24 ml, 1mmol) dans 5 ml de dichlorométhane. On laisse la température remonter à l'ambiante pendant 1 h. On chauffe ensuite au reflux du dichlorométhane pendant 2 h puis on ajoute 0,1 ml (1 mmol) d'acide trifluoacétique. On filtre le mélange réactionnel sur de la silice (10 g). On sèche le filtrat sur sulfate de magnésium puis après filtration sur papier, on évapore le solvant sous vide à température ambiante. On obtient 0,38 g d'un liquide incolore soit un rendement de 86 %.

RMN 'H (200,13 MHz, CDCl3)
S (ppm) = 2,49 (s, 3M, $SCH_3$).

RMN$^{19}$F : (188,35 MHz, CDCl3) $CF_3$ - $CF_2^f$ - $CF_2^e$ - $CF_2^d$ - $CF_2^c$ - $CF_2^b$-$CF_2^a$ - $COSCH_3$
S(ppm) = - 81,43 (t, $^hJ$ = 9,9 $H_2$, 3F, $CF_3$) ; - 116,97 (t, $^hJ$ = 13,2 $H_2$, 2F, $CF_2^a$)
;
- 121,87 (pic large, 2F, $CF_2^b$) ; - 122,51 (pic large, 4F,$CF_2^{c,d}$) ;
- 123,22 (pic large, 2F, $CF_2^e$) ; - 126,67 (pic large, 2F, $CF_2^f$).

RMN$^{13}$C (50,32 MHz, CDCl3)
S(ppm) = 11,86 (CH3) ; 107,67 à 120,96 (massifs non résolus, CFn) ; 187,13 (CO).

Spectre de masse (m/z, %)
m/z = 444 (M,2) ; 397 ($C_7$F15SCO, 10) ; 75 ($COSCH_3$, 100) ; 69 ($CF_3$, 31) ; M7 ($SCH_3$,45).

**EXEMPLE 12 :Greffage de Rf sur des silanes Insaturés**

*Mode opératoire général*

Dans une cuve cylindrique de 50 ml possédant 2 faces planes en quartz et munie d'un agitateur magnétique, on introduit 20 ml de dichlorométhane anhydre, ainsi que les réactifs (thioesters, substrat). On fait barboter de l'azote pendant 15 minutes. Le milieu réactionnel est ensuite soumis à une irradiation ultraviolette à 40°C.
L'avancement de la réaction est suivi par CPV. En fin d'irradiation, on additionne un étalon interne (trifluorométhoxybenzène) et une analyse RMN $^{19}$F est effectuée pour quantifier les différentes espèces fluorées.
Le dichlorométhane est ensuite évaporé sous vide de la trompe à eau sans chauffer.

**Cas du trifluorothioacétate d'octyle sur le silane cyclique insaturé 6**

On irradie pendant 2h20 à 40°C sous agitation une solution constituée de $CF_3COSC_8H_{17}$ (0,2 kg, 1 mmol) et de silane cyclique insaturé Cyclohexènyléthyl-Si$(CH_3)$(O-Et)$_2$ (0,24 g, 1 mmol) dans 20 ml de dichlorométhane anhydre.
On obtient une masse brute de 0,480 g dont aucun produit n'a été isolé du fait de la difficulté de séparation du disulfure d'avec les autres produits.
on obtient une trifluorométhylation en 3 et en 4 avec deux diastéréoisomères dans chaque position.

RMN$^{19}$ F (dosage, 138,35 MHz, CDCl3)

δ(ppm) = - 41,75 (s, 3F, $CF_3$S), CF3S$(CH_2)$ + $CH_3$, rdt = 10 %
- 72,67 (s large, 3F)
- 74,28 (d, $^rJ$ = 6,6 $H_2$, $CF_3$) rdt = 20 + 61
- 74,42 (d, $^rJ$ = 6,7 $H_2$, $CF_3$)
- 75,92 (s, 3F, $CF_3$ CO) $CF_3COSC_8H_{17}$, conversion = 96 %

Spectre de masse (m/z, %)

- * tr = 11.967 min (SCAN 309) (produit 9)
  m/z = 153 ($C_5H14O_2Sif$, 35) ; 133 ($C^5H_{13}SiO$, 100) ; 109 ($C_3H_{10}SiOF$, 13) ;
  89 ($C_3H_9SiO$, 15) ; 61 ($CH_5SiO$, 10).
- * tr = 12.127 min (SCAN 314) (produit 9)
  m/z = 153 ($C_5H14O_2Sif$, 37) ; 133 ($C^5H_{13}SiO$, 100) ; 109 ($C_3H_{10}SiOF$, 13) ;
  89 ($C_3H_9SiO$, 12) ; 61 ($CH_5SiO$, 10).

**EXEMPLE 13 :Cas du pentadécaluorothioocanoate de méthyle sur le silane cyclique insaturé 6**

Dans les mêmes conditions, on irradie la solution de $C_7F_{15}COSCH_3$ (0,44 g, 1 mmol) et de silane (0,24 g, 1 mmol) dans 20 ml de dichlorométhane anhydre pendant 5 h à 40 °C sans agitation. Les produits se dégradent partiellement sur la silice. Aucune purification n'a abouti.

C P V

Cette analyse révèle la formation de 4 produits isomères (selon la SM) groupés en 2 couples de produits.

tr = 0,61 min ; 10,22 % ; $C_7F_{15}SCH_3$
tr = 7,68 min ; 55 %
tr = 7,81 min ; 1,5 % 4 produits isomères
tr = 8,04 min ; 22,7 %
tr= 8,17 min ; 9,3%

RMN$^{19}$F (128,35 MHz, CDCl3)
Dosage avec $PhOCF_3$ comme étalon :

| | |
|---|---|
| $C_7F_{15}SCH_3$ | 9,8 % |
| $C_7F_{15}COSCH_3$ | 5,7 % rendement brut pour la somme des hs isomères = 68,5 % |
| Σ (RfCH) | 84 % |

Spectre de masse (m/z %)

Temps de rétention des isomères (en min) : 1h,55 ; 1h,73 ; 15,15 ; 15,41.
Pics communs aux 4 isomères
m/z = 460 ($C_{15}HF_{14}$, 1); 432 ($C_{13}M_{10}F14$, 1) ; 419 ($C_{12}H_8F_{14}$, 1);
153 ($C_5M_{14}0_2SiF$, 18) ; 133 ($C_5H_{13}SiO$, 100) ; 109 ($C_3H_{10}$ SiOF, 11) ;
89 ($C_3$ $H_9$ SiO, 12) ; 61 ($CH_5$ SiO, 4).

**EXEMPLE 14** :Greffage de $R_f$ sur un diène

**Cas du trifluorothioacétate d'éthyle sur le vinyl-4 cyclohexène 16**

On irradie une solution de $CF_3COSCH_2CH3$ (0,16 g, 1 mmol) et de 4-vinyl cyclohexène 16 (1,10 g, 1 mmol) dans 20 ml de dichlorométhane anhydre pendant 5h20 à 40°C sans agitation.

Dosage par RMN$^{19}$ F (188,35 MH2, $CDcl_3$)

S (ppm) = - 64,25 (m), 18 (*), rdt 6,9 %
- 66,71 ($t^3$,J = 10,9$H_2$), 20 (**) , rdt 30 %
- 72,15 (m) , 17 (?), rdt 4,2 %
- 72,80 (d, intensité $I^1$)

(*) par analogie avec S = - 64,7 ppm pour trans $CF_3CH = CHC_5H_{11}$ (C.J. Bicokes, P.L. Coe, A.E. Pedder, J.C. Tatlow, J. Chem, Soc, Perkin Trans. I, 1978, 202).

- 72,98 (d, intensité $I^1$) 19 (***), rdt 1,6 %
- 74,1 (d, intensité $I^2$)
- 74,26 (d, intensité $I^2/2$) 19 (***), rdt 4,2 %

En RMN$^{19}$F, le rapport de trifluorométhylation entre la double liaison exo et la double liaison endocyclique est grossièrement estimé à 3/1.
En RMN1H, le rapport entre les protons vinyliques exo et les protons vinyliques endocycliques est de l'ordre de 2,3 et le rapport entre les motifs $CH_2CF_3$ et $CHCF_3$ se situe aux alentours de 2.

Par microdistillation, on obtient une goutte de produit analysé en RMN1H$^{19}$,F. Les spectres sont analogues à ceux dus produit brut mais la très grande dilution de l'échantillon ne permet pas d'affiner l'analyse.

On a également tenté de chromatographier le produit (masse silice = 6 g, quantité de produit mise en jeu 130 mg) en éluant avec l'éther de pétrole. Les conditions semblent adaptées à la séparation du mélange, néanmoins la très faible quantité de produit récupéré dans chaque fraction ne permet pas d'identification correcte.

**EXEMPLE 15 :Synthèse de pentadécafluoroheptyl octyl thioéther $C_7F_{15}SC_8H_{17}$ 22**

***a) En l'absence de disulfure***

On irradie une solution de $C_7F_{15}COSC_8H_{17}$ (0,55 g, 1 mmol) dans 20 ml de dichlorométhane anhydre pendant 3h20 à 40 °C sans agitation.
RMN$^{19}$F (188,35 MHz, CDCl3)

S (ppm) = - 87,69 (t,$^4$J = 12,5 $H_2$, 2F, SC<u>F</u>$_2$) rdt = 24 % → $C_7$F15S$C_8H_{17}$
- 116,78 (t,4J = 13,2 $H_2$, 2F, C<u>F</u>$_2$COS)conversion = 67 % → $C_7F_{15}COSC_8H_{17}$
- 137,8 (d,$^2$J = 49,8$H_2$, 2F, $CF_2$H) rdt = 28 % → $C_6F_{13}$-$CF_2H$

***B) En présence de disulfure***

On irradie une solution de $C_7F_{15}COSC_8H_{17}$ (0,55 g, 1 mmol) et de ($C_8H_{17}$S (0,3 g, 1 mmol) dans 20 ml de dichlorométhane anhydre pendant 3h15mn à 40°C sans agitation.
RMN$^{19}$F (188,35 MHz, CDCl3)

S (ppm) = - 87,76 (t,$^4$J = 12 $H_2$, 2F, SC<u>F</u>$_2$) rdt = 51 % → $C_7$F15S$C_8H_{17}$
- 116,98 (t,$^4$J = 13,2 $H_2$, 2F, C<u>F</u>$_2$CO) conversion = 80 % → $C_7F_{15}COSC_8H_{17}$
- 137,4 (t,$^2$J = 49,8$H_2$, 2F, C<u>F</u>$_2$H) rdt = 20 % → $C_6F_{13}$-$CF_2H$

Après optimisation de cette réaction, au niveau de la durée d'irradiation, on aboutit à 5h45 min, à 40°C sans agitation, à un rendement brut de 57 % en $C_7F_{15}SC_8H_{17}$ avec disparition du RfH? ($C_6F_{13}CF_2H$).

RMN$^{19}$F de $C_7F_{15}SC_8H_{17}$ $CF_3$ $CF_2^f$ $CF_2^e$ $CF_2^d$ $CF_2^c$ $CF_2^b$ $CF_2^a$S$C_8H_{17}$

S (ppm) = - 81,09 (t, $^h$J = 9 $H_2$, 3F, C<u>F</u>$_3$) ; - 87,68 (t,$^h$J = 12 H2, 2F, SC<u>F</u>2) ; - 120,8 (pic large, 2F, C<u>F</u>2$^b$) ; - 121,46 (pic large, 2 F, C<u>F</u>2$^c$) ; - 122,22 (pic large, 2 F C<u>F</u>$_2$$^d$) ; - 122,96 (pic large, 2F, C<u>F</u>$_2$$^e$) ; - 126,37 (pic large, 2 F, C<u>F</u>$_2$$^f$).

**EXEMPLE 16 Synthèse de pentadécafluoroheptyl méthyl thioéther $C_7F_{15}SCH_3$ 23**

***a) En l'absence de disulfure***

On irradie une solution de $C_7F_{15}COSCH_3$ (0,44 g, 1 mmol) dans 20 ml de dichlorométhane anhydre pendant 4h37 à 40 °C sans agitation.

<u>Dosage RMN$^{19}$F</u> du mélange brut (128,35 MHz, CDCl3)

S (ppm) = - 91,18 (t,$^4$J = 13,5 $H_2$, 2F, SC<u>F</u>$_2$) rdt = 53 % (absence de C<u>F</u>$_2$CO et de $CF_2$4)

Par microdistillation à pression atmosphérique, on obtient quelques gouttes d'un liquide jaune clair pur en RMN1H et $^{19}$F.

RMN$^{19}$F (188,35 MHz, CDCl3) $CF_3$ $CF_2^f$ $CF_2^e$ $CF_2^d$ $CF_2^c$ $CF_2^b$ $CF_2^a$ $SCH_3$

S (ppm) = - 81,29 (t,$^4$J = 8,5 $H_2$, 3F, $CF_3$) ; - 90,92 (t,$^h$J = 14$H_2$, 2F, $SCF_2$) ; - 120,35 (pic large, 2F, $C\underline{F_2}^b$) ; - 122,05 (pic large, 2F, $C\underline{F_2}^c$) ; - 122,73 (pic large, 2F,$C\underline{F_2}^d$) ; - 123,46 (pic large, 2F,$C\underline{F_2}^e$) ; - 126,87 (pic large, 2F,$C\underline{F_2}^f$).

RMN1H (200,13 MHz, CDCl3)
S (ppm) = 2,4 (s, 3H, $SC\underline{H_3}$).

***a) En présence de disulfure***

On irradie une solution de $C_7F_{15}COSCH_3$ (0,44 g, 1 mmol) et de disulfure ($CH_3S$-S-$CH_3$) (0,09 g, 1 mmol) dans 20 ml de dichlorométhane anhydre à 40°C sous agitation pendant 4h50.

RMN$^{19}$F dosage du mélange brut (188,35 MHz, CDCl3)

S (ppm) = - 90,67 (t,$^h$J = 13,2 $H_2$, 2F, $SC\underline{F_2}$) rdt = 81 % - 116,79 (t,$^h$J = 9 $H_2$, 2F, $C\underline{F_2}CO$) conversion = 95 % → $C_7F_{15}COSCH_3$.

Après évaporation du solvant, on purifie le résidu par chromatographie éclair sur silice (10 g) à partir de 0,332 g de produit. L'éluant utilisé est l'éther de pétrole. Après évaporation des fractions à 60-70° sous pression atmosphérique, on isole 0,110 g de $C_7F_{15}SCH_3$ pur en RMN1H et $^{19}$F, représentant un rendement de 40 %. L'utilisation de pentane comme éluant permettra probablement d'obtenir un rendement plus élevé en produit isolé.

**EXEMPLE 17 : Photolyse du thioloester**

On a photolysé l'éthylxanthate de S-trifluoroacétyle dans la masse, sans solvant :

$$CF_3\text{-}CO\text{-}S\text{-}CS\text{-}OCH_2CH_3 \xrightarrow{h\nu\ (\text{tungstène, 500W})} CF_3\text{-}CS\text{-}OCH_2CH_3\ (65\ \%) + CO$$

On a essayé de photolyser ce thiolester dans les conditions mises au point précédemment, c'est-à-dire en solution dans le dichlorométhane :

$$CF_3\text{-}CO\text{-}S\text{-}CS\text{-}OCH_2CH_3 \xrightarrow{h\nu\ /CH2Cl2\ /40°C/\ 50\ min} CF_3\text{-}CS\text{-}OCH_2CH_3\ (25\ \%) + CO$$

**EXEMPLE 18 : Photolyse de l'éthylxanthate de S-trifluoroacétyle en présence de 1-dodécène**

*Essai en solution*

Une solution d'éthylxanthate de S-trifluoroacétyle (0,22 g, 1 mmol) et de 1-dodécène (0,17 g, 1 mmol) dans 20 ml de dichlorométhane anhydre, contenue dans un réacteur cylindrique en verre pyrex de caractéristiques identiques à celui en quartz, est irradiée à 40°C (lampe HPK 125) :

$$CF_3\text{-}CO\text{-}S\text{-}CS\text{-}OEt + CH_2\text{=}CH\text{-}R \xrightarrow{h\nu\ /CH2Cl2\ /40°C/\ 80min} CF_3\text{-}S\text{-}CS\text{-}OEt\ (23\ \%) + CO +$$
$$CF_3\text{-}CH_2\text{-}CH(\text{-}R)\text{-}S\text{-}CS\text{-}OEt\ (12\ \%) + CF_3\text{-}CH_2\text{-}CH_2\text{-}R\ (2\ \%) + (Et0\text{-}CS\text{-}S\text{-})_2$$

En solution, la cis-addition formelle de $CF_3$-SC(S)OEt sur le 1-dodécène demeure minoritaire (12 %), mais l'hydrotrifluorométhylation de cette oléfine est réduite à seulement 2 %.

Le thioloester considéré est donc bien clivé photochimiquement et la décarbonylation du radical trifluoroacétyle engendré conduit là encore au radical trifluorométhyle. On ne retrouve cependant que 40 % environ du motif $CF_3$ engagé, majoritairement dans le produit de décarbonylation formelle du réactif initial (RR = 23 %). La majeure partie du radical trifluorométhyle est sous forme de composés gazeux comme $C_2F_6$ $CF_3H$ ou $CF_3Cl$.

*Essai en l'absence de solvant et en deux temps*

$$CF_3\text{-CO-S -CS-OEt} \xrightarrow{h\gamma(\text{lampe W})} CF_3\text{-S-CS-OEt}$$

$$CF_3\text{-S-CS-OEt} + CH_2\text{=CH-R} \xrightarrow{h\gamma(\text{lampe W})/20h} CF_3\text{-}CH_2\text{-CH(-R)-S-CS-OEt (65 \%)}$$

Cet essai a conduit au résultat escompté, sous réserve d'opérer en deux étapes. Dans la première étape, on procède à la décarbonylation photochimique du réactif $CF_3COS\text{-}C(S)OEt$ et, dans la seconde étape, on photolyse le produit résultant en présence d'oléfine.

Le rendement de cette seconde réaction est de 65 %.

## Revendications

1. Réactif, **caractérisé par le fait qu'**il comporte pour addition successive ou simultanée :

   - un composé de formule (I):

     Rf-M(X)(Z)n-Y-R ;

     avec R représentant un radical carboné, avantageusement d'au plus 15, de préférence 10 atomes de carbone, de préférence choisi parmi les alcoyles, les aryles, les acyles, y compris les hydrocarbylchalcogénylcarbonyles, et les thioacyles, y compris hydrocarbylchalcogénylthiocarbonyle;
     avec $R_f$ représentant un radical de formule :

     $$[R_2\text{-}(C\Xi_2)_p\text{-}][R_1\text{-}(C\Xi_2)_m\text{-}]CF\text{-}$$

     où $R_1$ et $R_2$, semblables ou différents, représentent un atome d'halogène léger, fluor ou de chlore, de préférence fluor, un radical carboné
     où m est zéro ou un entier choisi dans l'intervalle fermé allant de 0 à 12;
     où p est zéro ou un entier choisi dans l'intervalle fermé allant de 0 à 12 ;
     où les Ξ semblables ou différents représentent des radicaux perhalogénés, avantageusement perfluorés, des atomes de chlore ou de préférence de fluor ; avec la condition que lorsque m et/ou p sont égaux à zéro, $R_1$ et/ou $R_2$ sont électroattracteurs, avantageusement un atome de fluor ou de chlore, de préférence un atome de fluor.

     avec n représentant zéro ou 1 ;
     avec M représentant un métalloïde choisi parmi le carbone et les chalcogènes de rang supérieur à l'oxygène ;
     avec X, Y, Z semblables ou différents représentant un chalcogène avec la condition que X, Y et, quand n est différent de zéro, Z ne soient pas tous oxygène;

   - une source de radicaux choisis parmi les radicaux issus de l'activation actinique ou de la thermolyse des composés de formule I ou issus d'un initiateur chimique, avantageusement choisis parmi les peroxydes, les dérivés azoïques et les stannanes.

2. Réactif selon la revendication 1, **caractérisé par le fait que** Rf représentant un radical de formule (II) :

   R1-(CΞ2)m-CF2-

   où R1 est un atome de fluor (ou de chlore) ou un radical carboné
   où m est zéro ou un entier compris entre 0 et 12 ;

où les Ξ semblables ou différents représentent des atomes de chlore ou de préférence de fluor ;
avec la condition que lorsque m est égal à zéro, R1 est électroattracteur, de préférence un atome de fluor ou de chlore.

**3.** Réactif selon les revendications 1 et 2, **caractérisé par le fait que** ledit la source de radicaux est le composé de formule (I) lui-même, soumis à une source actinique, avantageusement de longueur d'onde compris entre 200 et 800 nm.

**4.** Réactif selon les revendications 1 à 3, **caractérisé par le fait que** ladite source de radicaux est un tiers composé chimique donnant naissance à des radicaux libres et choisi parmi les peroxydes, les dérivés azoïques et les stannanes.

**5.** Réactif selon les revendications 1 à 4, **caractérisé par le fait qu'**il comporte en outre un solvant.

**6.** Réactif selon les revendications 1 à 5, **caractérisé par le fait que** Y est choisi parmi le sélénium le soufre et le tellure, avantageusement choisi parmi le sélénium et le soufre, de préférence un soufre.

**7.** Réactif selon les revendications 1 à 6, **caractérisé par le fait que** X est choisi parmi l'oxygène et le soufre, de préférence l'oxygène.

**8.** Réactif selon les revendications 1 à 7, **caractérisé par le fait que** Z est choisi parmi l'oxygène et le soufre, de préférence l'oxygène.

**9.** Réactif selon les revendications 1 à 8, **caractérisé par le fait que** M est un atome de carbone, n est zéro, X est oxygène et Y est soufre ou sélénium.

**10.** Réactif selon les revendications 1 à 8, **caractérisé par le fait que** M est un atome de soufre ou de sélénium, n est un, X est oxygène et Y est soufre ou sélénium.

**11.** Composé de formule (I)

$$Rf\text{-}M(X)(Z)n\text{-}Y\text{-}R \;;$$

avec R représentant un radical carboné, avantageusement d'au plus 15, de préférence 10 atomes de carbone, choisi parmi les acyles, y compris les hydrocarbylchalcogénylcarbonyles, et les thioacyles, y compris hydrocarbylchalcogénylthiocarbonyles;
avec $R_f$ représentant un radical de formule :

$$[R_2\text{-}(C\Xi_2)_p\text{-}][R_1\text{-}(C\Xi_2)_m\text{-}]CF\text{-}$$

où $R_1$ et $R_2$, semblables ou différents, représentent un atome d'halogène léger, fluor ou de chlore, de préférence fluor, un radical carboné
où m est zéro ou un entier choisi dans l'intervalle fermé allant de 0 à 12 ;
où p est zéro choisi dans l'intervalle fermé allant de 0 à 12 ;
où les Ξ semblables ou différents représentent des radicaux perhalogénés, avantageusement perfluorés, des atomes de chlore ou de préférence de fluor ; avec la condition que lorsque m et/ou p sont égaux à zéro, $R_1$ et/ou $R_2$ sont électroattracteurs, avantageusement un atome de fluor ou de chlore, , de préférence un atome de fluor.

Avec n représentant zéro ou 1 ;
avec M représentant un métalloïde choisi parmi le carbone et les chalcogènes de rang supérieur à l'oxygène ;
avec Y représentant un soufre ou un sélénium ;
avec X, Z semblables ou différents représentant un chalcogène.

**12.** Composé de formule (I).

Rf-M(X)(Z)n-Y-R ;

avec R représentant un radical carboné, avantageusement d'au plus 15, de préférence 10 atomes de carbone, de préférence choisi parmi les alcoyles, les aryles, les acyles, y compris les hydrocarbylchalcogénylcarbonyles, et les thioacyles, y compris hydrocarbylchalcogénylthiocarbonyle;
avec $R_f$ représentant un radical de formule :

$$[R_2\text{-}(C\Xi_2)_p\text{-}][R_1\text{-}(C\Xi_2)_m\text{-}]CF\text{-}$$

où $R_1$ et $R_2$, semblables ou différents, représentent un atome d'halogène léger, fluor ou de chlore, de préférence fluor, un radical carboné
où m est zéro ou un entier choisi dans l'intervalle fermé allant de 0 à 12;
où p est zéro ou un entier choisi dans l'intervalle fermé allant de 0 à 12;
où les Ξ semblables ou différents représentent des radicaux perhalogénés, avantageusement perfluorés, des atomes de chlore ou de préférence de fluor ; avec la condition que lorsque m et/ou p sont égaux à zéro, $R_1$ et/ou $R_2$ sont électroattracteurs, avantageusement un atome de fluor ou de chlore, , de préférence un atome de fluor.

avec n représentant zéro ou 1 ;
avec M représentant un métalloïde choisi parmi le carbone et les chalcogènes de rang supérieur à l'oxygène ;
avec X, Y, Z semblables ou différents représentant un chalcogène ;

**caractérisé par le fait que** $R_f$ comporte plus de trois carbones

**13.** Composé selon les revendications 11 et 12, **caractérisé par le fait que** Rf est un radical carboné d'au plus 20 carbones, de préférence d'au plus 15 carbones.

**14.** Procédé de traitement de composé présentant au moins une fonction nucléophile, **caractérisé par le fait qu'**il comporte au moins une étape dans laquelle on met ledit composé présentant au moins une fonction nucléophile, en contact avec composé de formule (I):

Rf-M(X)(Z)n-Y-R ;

avec R représentant un radical carboné, avantageusement d'au plus 15, de préférence 10 atomes de carbone, de préférence choisi parmi les alcoyles les aryles, les acyles, les thioacyles ;
avec $R_f$ représentant un radical de formule :

$$[R_2\text{-}(C\Xi_2)_p\text{-}][R_1\text{-}(C\Xi_2)_m\text{-}]CF\text{-}$$

où $R_1$ et $R_2$, semblables ou différents, représentent un atome d'halogène léger, fluor ou de chlore, de préférence fluor, un radical carboné
où m est zéro ou un entier choisi dans l'intervalle fermé allant de 0 à 12;
où p est zéro ou un entier choisi dans l'intervalle fermé allant de 0 à 12;
où les Ξ semblables ou différents représentent des radicaux perhalogénés, avantageusement perfluorés, des atomes de chlore ou de préférence de fluor ; avec la condition que lorsque m et/ou p sont égaux à zéro, $R_1$ et/ou $R_2$ sont électroattracteurs, avantageusement un atome de fluor ou de chlore, , de préférence un atome de fluor.

avec n représentant zéro ou 1 ;
avec M représentant un métalloïde choisi parmi le carbone et les chalcogènes de rang supérieur à l'oxygène ;
avec X, Y, Z semblables ou différents représentant un chalcogène avec la condition que X, Y, Z ne soient pas tous oxygène quand n est différent de zéro; ledit contact ion étant réalisé avec initiation radicalaire au moyen d'une source actinique, ou d'un initiateur radicalaire chimique.

**15.** Procédé selon la revendication 14, **caractérisé par** le fait l'on met en oeuvre une réaction radicalaire de transformation du composé I **(I)** en thioéther perhalogénoalcoylé en l'absence de substrat.

**16.** Procédé selon la revendication 14, **caractérisé en ce qu'**il consiste essentiellement à faire réagir par voie radicalaire le composé **(I)** avec au moins un substrat.

**17.** Procédé selon l'une quelconque des revendications 14 et 16, **caractérisé par le fait que** la réaction consiste en une photolyse d'un composé **(I)** en présence d'un substrat dans un milieu réactionnel comprenant éventuellement un solvant organique, à une longueur d'onde correspondant au maximum d'absorption du groupe chromophore.

**18.** Procédé selon la revendication 17, **caractérisé par le fait que** l'on choisit entre 200 et 800 nm, de préférence entre 210 et 600 nm.

**19.** Procédé selon les revendications 17 et 18, **caractérisé par le fait que** le solvant est choisi de telle sorte qu'il possède une transparence supérieure ou égale à 50 %, de préférence à 70 % et, plus préférentiellement encore, à 90 % à la longueur d'onde .

**20.** Procédé selon la revendication 19, **caractérisé par le fait que** la longueur d'onde est de l'ordre de 210 - 600 nm et en ce que le solvant est sélectionné de préférence parmi les composés suivants : acétonitrile ; éthanol ; butanol ; dichlorométhane ; cyclohexane ; cyclopentane ; dichlorol,2éthane ; diméthyl-2,2butane ; n-heptane ; n-hexane ; méthanol ; méthyl-2butane ; iso-octane ; n-pentane ; propanol-2 ; trichloro-1,2,2trifluoroéthane ; trifluoro-2,2,2éthanol, ou un mélange de ceux-ci.

**21.** Procédé selon l'une quelconque des revendications 17 à 20, **caractérisé par le fait que** l'on prévoit une température $T_R$ inférieure ou égale à la température d'ébullition, à pression donnée, du solvant sélectionné.

**22.** Procédé selon la revendication 21, **caractérisé par le fait que** les composés visés sont les composés perhalogéno en particulier perfluoro-alcoylés et/ou -S-alcoylés et en ce que $T_R$ est supérieure ou égale à 20° C, de préférence à 35°C et, plus préférentiellement encore, de l'ordre de 40° C.

**23.** Procédé selon la revendication 21, **caractérisé par le fait que** les composés visés sont les composés perhalogéno, en particulier perfluoro-acylés, et en ce que $T_R$ est inférieure ou égale à 30° C, de préférence à 25° C et, plus préférentiellement encore, est de l'ordre de 20° C.

**24.** Procédé selon l'une quelconque des revendications 14 à 23, **caractérisé par le fait que** le substrat mis en oeuvre répond à la formule suivante :

R'-S-S-R" **(IV)**

où les radicaux R', R" du composé (IV) et le radical R du composé (I) peuvent être identiques ou différents entre eux. Ils sont de préférence identiques et répondent à la définition de R donnée ci-dessus.

**25.** Procédé selon l'une des revendications 14 à 11 **caractérisé par le fait que** le substrat mis en oeuvre est, au moins partiellement, organique, de préférence insaturé et, plus préférentiellement encore, sélectionné parmi les (cyclo) alcènes, les (cyclo) alcynes, les aromatiques ou leur mélanges.

**26.** Procédé selon la revendication 25, **caractérisé par le fait que** le substrat est formé par au moins l'un des produits appartenant à au moins l'une des classes chimiques suivantes :

- alcènes et alcynes non fonctionnalisés sur l'insaturation,
- éthers et esters d'énols,
- perhalogénocarboxylates d'énols,
- sulfures de vinyle,
- énamines,
- éneoxy, énethio et énéaminostannanes,
- allystannanes,
- éneoxy, énethio et éneaminosilanes,

- allylsilanes.

**27.** Procédé selon l'une quelconque des revendications 14 à 26, **caractérisé par le fait que** l'on choisit le ratio molaire thioloester **(I)**/substrat compris entre 0,1 et 10, de préférence entre 0,5 et 2 et plus préférentiellement encore entre 0,9 et 1,1.

**28.** Procédé selon l'une quelconque des revendications 14 à 27, **caractérisé par le fait que** la réaction consiste en une thermolyse.

**29.** Procédé selon la revendication 28, **caractérisé par le fait que** la température de thermolyse est comprise entre 350 et 650° C, de préférence entre 400 et 600° C.

**30.** Procédé selon l'une quelconque des revendications 28 et 29, **caractérisé par le fait que** la thermolyse est réalisée dans un réacteur tubulaire en verre ou en quartz.

**31.** Dérivé perfluoroalcoylé de formule VI :

$$(R_4)(R_5)CH — C(\mathbf{Rf})(R_6)(R_7)(VI)$$

**caractérisé par le fait qu'**il est susceptible d'être obtenu selon le procédé selon l'une des revendications précédentes à partir d'un substrat de formule (V).

$$(R_4)(R_5)C{=}C(R_6)(R_7)$$

avec $R_4$, $R_5$, $R_6$, $R_7$ semblables ou différents choisis parmi l'hydrogène, les radicaux hydrocarbonés et notamment parmi les alcoyles, y compris les cycloalcoyles et les aralcoyles, et parmi les aryles avec la condition que au moins un des $R_4$, $R_5$, $R_6$, $R_7$ représente un radical monovalent portant un atome de silicium éloigné de la valence libre d'au moins de un, avantageusement d'au moins deux, atomes de carbone .

**Patentansprüche**

**1.** Reagenz, **dadurch gekennzeichnet, daß** es für aufeinanderfolgende oder gleichzeitige Addition enthält:

- eine Verbindung der Formel (I):

$$R_f\text{-}M(X)(Z)_n\text{-}Y\text{-}R$$

in der R einen Kohlenstoff enthaltenden Rest mit vorzugsweise höchstens 15, insbesondere 10 Kohlenstoffatomen bedeutet, vorzugsweise gewählt aus den Alkylen, Arylen, Acylen einschließlich den Hydrocarbylchalkogenylcarbonylen, den Thioacylen einschließlich Hydrocarbylchalkogenylthiocarbonylen;
in der $R_f$ einen Rest der Formel

$$[R_2\text{-}(C\Xi_2)_p\text{-}][R_1\text{-}(C\Xi_2)_m\text{-}]CF\text{-}$$

bedeutet,
in der $R_1$ und $R_2$ gleich oder verschieden ein leichtes Halogen, Fluor oder Chlor, vorzugsweise Fluor, oder einen Kohlenstoff enthaltenden
Rest bedeuten,
in der m gleich 0 oder eine ganze Zahl in dem abgeschlossenen Intervall von 0 bis 12 ist;
in der p gleich 0 ist oder eine ganze Zahl in dem abgeschlossenen Intervall von 0 bis 12 ist;
in der die Ξ gleiche oder verschiedene perhalogenierte, vorteilhaft perfluorierte Reste, Chlor- oder vorzugsweise Fluoratome bedeuten, unter der Bedingung, daß $R_1$ und $R_2$ elektronenanziehend, vorteilhafterweise ein Fluor- oder Chloratom, vorzugsweise ein Fluoratom sind, wenn m und/oder p gleich 0 sind;

mit n gleich 0 oder 1;
mit M als Metalloid, ausgewählt unter Kohlenstoff und den ranghöheren Chalkogenen als Sauerstoff;
mit X, Y, Z, die gleich oder verschieden ein Chalkogen bedeuten unter der Bedingung, daß X, Y und; wenn n ungleich 0 ist, auch Z nicht alle Sauerstoff sein dürfen;

- eine Quelle für Radikale, die unter den durch Strahlungsaktivierung oder Thermolyse von Verbindungen der Formel I gebildeten Radikalen oder einen chemischen Initiator, insbesondere den Peroxiden, den Azoverbindungen und den Stannanen ausgewählt sind.

**2.** Reagenz nach Anspruch 1, **dadurch gekennzeichnet, daß** $R_f$ ein Rest der Formel (II) ist:

$$R_1\text{-}(C\Xi 2)_m\text{-}CF_2$$

in der $R_1$ ein Fluor- (oder Chlor-)atom oder ein kohlenstoffhaltiger Rest ist;
in der m gleich 0 oder eine ganze Zahl zwischen 0 und 12 ist;
in der die Ξ gleich oder verschieden Chlor- oder vorzugsweise Fluoratome bedeuten unter der Bedingung, daß $R_1$ elektronenanziehend, vorzugsweise ein Fluor- oder Chloratom ist, wenn m gleich 0 ist.

**3.** Verfahren nach den Ansprüchen 1 und 2, **dadurch gekennzeichnet, daß** die Radikalquelle die Verbindung der Formel (I) selbst ist, die einer Strahlungsquelle, vorteilhaft einer Wellenlänge von 200 bis 800 nm ausgesetzt wurde.

**4.** Reagenz nach den Ansprüchen 1 bis 3, **dadurch gekennzeichnet, daß** die genannte Quelle für Radikale eine dritte chemische Verbindung ist, die freie Radikale liefert und die unter den Peroxiden, den Azoverbindungen oder den Stannanen gewählt ist.

**5.** Reagenz nach den Ansprüchen 1 bis 4, **dadurch gekennzeichnet, daß** es zusätzlich ein Lösungsmittel enthält.

**6.** Reagenz nach den Ansprüchen 1 bis 5, **dadurch gekennzeichnet, daß** Y unter Selen, Schwefel und Tellur, vorteilhafterweise unter Selen und Schwefel und vorzugsweise als Schwefel gewählt ist.

**7.** Reagenz nach den Ansprüchen 1 bis 6, **dadurch gekennzeichnet, daß** X unter Sauerstoff und Schwefel, vorzugsweise als Sauerstoff, gewählt ist.

**8.** Reagenz nach den Ansprüchen 1 bis 7, **dadurch gekennzeichnet, daß** Z unter Sauerstoff und Schwefel, vorzugsweise als Sauerstoff, gewählt ist.

**9.** Reagenz nach den Ansprüchen 1 bis 8, **dadurch gekennzeichnet, daß** M ein Kohlenstoffatom, n gleich 0, X Sauerstoff und Y Schwefel oder Selen ist.

**10.** Reagenz nach den Ansprüchen 1 bis 8, **dadurch gekennzeichnet, daß** M ein Schwefel- oder Selenatom, n gleich 1, X Sauerstoff und Y Schwefel oder Selen ist.

**11.** Verbindung der Formel (I)

$$R_f\text{-}M(X)(Z)_n\text{-}Y\text{-}R$$

in der R einen Kohlenstoff enthaltenden Rest mit vorzugsweise höchstens 15, insbesondere 10 Kohlenstoffatomen, vorzugsweise gewählt aus den Alkylen, Arylen, Acylen einschließlich den Hydrocarbylchalkogenylcarbonylen, den Thioacylen einschließlich Hydrocarbylchalkogenylthiocarbonylen bedeutet;
in der $R_f$ einen Rest der Formel

$$[R_2\text{-}(C\Xi_2)_p\text{-}][R_1\text{-}(C\Xi_2)_m\text{-}]CF\text{-}$$

bedeutet,
in der $R_1$ und $R_2$ gleich oder verschieden ein leichtes Halogen, Fluor oder Chlor, vorzugsweise Fluor, oder

einen Kohlenstoff enthaltenden Rest bedeuten;
in der m gleich 0 oder eine ganze Zahl in dem abgeschlossenen Intervall von 0 bis 12 ist;
in der p gleich 0 ist oder eine ganze Zahl in dem abgeschlossenen Intervall von 0 bis 12 ist;
in der die Ξ gleiche oder verschiedene perhalogenierte, vorteilhaft perfluorierte Reste, Chlor- oder vorzugsweise Fluoratome bedeuten, unter der Bedingung, daß $R_1$ und $R_2$ elektronenanziehend, vorteilhafterweise ein Fluor- oder Chloratom, vorzugsweise ein Fluoratom sind, wenn m und/oder p gleich 0 sind;
mit n gleich 0 oder 1;
mit M als Metalloid, ausgewählt unter Kohlenstoff und den ranghöheren Chalkogenen als Sauerstoff;
mit Y, das Schwefel oder Selen bedeutet;
X und Z gleich oder verschieden ein Chalkogen bedeuten.

**12.** Verbindung der Formel (I)

$$R_f\text{-}M(X)(Z)_n\text{-}Y\text{-}R$$

in der R einen kohlenstoffhaltigen Rest mit vorzugsweise höchstens 15, insbesondere 10 Kohlenstoffatomen bedeuten, vorzugsweise ausgewählt unter den Alkylen, den Arylen, den Acylen einschließlich den Hydrocarbylchalkogenylcarbonylen, den Thioacylen einschließlich Hydrocarbylchalkogenylthiocarbonylen;
mit $R_f$ das einen Rest der Formel

$$[R_2\text{-}(C\Xi_2)_p\text{-}][R_1\text{-}(C\Xi_2)_m\text{-}]CF\text{-}$$

bedeutet,
in der $R_1$ und $R_2$ gleich oder verschieden ein leichtes Halogen, Fluor oder Chlor, vorzugsweise Fluor, oder einen Kohlenstoff enthaltenden Rest bedeuten;
in der m gleich 0 oder eine ganze Zahl in dem abgeschlossenen Intervall von 0 bis 12 ist;
in der p gleich 0 ist oder eine ganze Zahl in dem abgeschlossenen Intervall von 0 bis 12 ist;
in der die Ξ gleiche oder verschiedene perhalogenierte, vorteilhaft perfluorierte Chlor- oder vorzugsweise Fluoratome bedeuten, unter der Bedingung, daß $R_1$ und $R_2$ elektronenanziehend, vorteilhafterweise ein Fluor- oder Chloratom, vorzugsweise ein Fluoratom sind, wenn m und/oder p gleich 0 sind;
mit n gleich 0 oder 1;
mit M als Metalloid ausgewählt unter Kohlenstoff und den ranghöheren Chalkogenen als Sauerstoff;
mit gleichen oder verschiedenen X, Y, Z, die ein Chalkogen bedeuten,

**dadurch gekennzeichnet, daß** $R_f$ mehr als drei Kohlenstoffatome enthält.

**13.** Verbindung nach den Ansprüchen 11 oder 12, **dadurch gekennzeichnet, daß** $R_f$ ein kohlenstoffhaltiger Rest mit höchstens 20, vorzugsweise höchstens 15 Kohlenstoffatomen ist.

**14.** Verfahren zur Behandlung einer Verbindung, die wenigstens eine nukleophile Funktion aufweist, **dadurch gekennzeichnet, daß** es wenigstens eine Stufe enthält, in der man die genannte Verbindung, die wenigstens eine nukleophile Funktion enthält, mit einer Verbindung der Formel (I) in Berührung bringt:

$$R_f\text{-}M(X)(Z)_n\text{-}Y\text{-}R$$

in der R einen kohlenstoffhaltigen Rest mit vorteilhaft höchstens 15, vorzugsweise 10 Kohlenstoffatomen, vorzugsweise ausgewählt aus den Alkylen, den Arylen, den Acylen und den Thioacylen;
in der $R_f$ einen Rest der Formel:

$$[R_2\text{-}(C\Xi_2)_p\text{-}][R_1\text{-}(C\Xi_2)_m\text{-}]CF\text{-}$$

darstellt, in der
$R_1$ und $R_2$ gleich oder verschieden ein leichtes Halogenatom, Fluor oder Chlor, vorzugsweise Fluor, und einen kohlenstoffhaltigen Rest bedeuten;

in der m gleich 0 oder eine ganze Zahl im abgeschlossenen Bereich von 0 bis 12 ist;
in der p gleich 0 oder eine ganze Zahl ist, in dem abgeschlossenen Bereich von 0 bis 12 ist;
in der die Ξ gleiche oder verschiedene perhalogenierte, vorzugsweise perfluorierte Reste, Chlor- oder vorzugsweise Fluoratome bedeuten, unter der Bedingung, daß $R_1$ und/oder $R_2$ elektronenanziehend sind, vorzugsweise ein Chlor- oder Fluoratom, insbesondere ein Fluoratom bedeuten, wenn n und/oder p gleich 0 sind;
in der n gleich 0 oder 1 ist;
in der M ein unter Kohlenstoff und den Chalkogenen mit höherem Rang als Sauerstoff ausgewähltes Metalloid bedeutet;
in der gleiche oder verschiedene X, Y, Z, ein Chalkogen bedeuten mit der Bedingung, daß X, Y, Z nicht alle Sauerstoff sind, wenn n verschieden von 0 ist, wobei das Inberührungbringen unter Auslösung durch Radikale mit Hilfe einer Strahlungsquelle oder einem chemischen Radikalbildner durchgeführt wird.

**15.** Verfahren nach Anspruch 14, **dadurch gekennzeichnet, daß** man eine radikalische Umwandlung der Verbindung (I) zu einem Perhalogenalkylthioether in Abwesenheit eines Substrats durchführt.

**16.** Verfahren nach Anspruch 14, **dadurch gekennzeichnet, daß** es im wesentlichen darin besteht, daß man die Verbindung (I) mit wenigstens einem Substrat umsetzt.

**17.** Verfahren nach irgendeinem der Ansprüche 14 und 16, **dadurch gekennzeichnet, daß** die Reaktion aus der Photolyse einer Verbindung (I) in Gegenwart eines Substrats in einem Reaktionsmilieu besteht, das gegebenenfalls ein organisches Lösungsmittel enthält, bei einer Wellenlänge, die dem Absorptionsmaximum der chromophoren Gruppe entspricht.

**18.** Verfahren nach Anspruch 17, **dadurch gekennzeichnet, daß** man die Wellenlänge zwischen 200 und 800 nm, vorzugsweise 210 und 600 nm, wählt.

**19.** Verfahren nach den Ansprüchen 17 und 18, **dadurch gekennzeichnet, daß** das Lösungsmittel so ausgewählt ist, daß es bei der Wellenlänge eine Transparenz gleich oder größer von 50 %, vorzugsweise über 70 %, und ganz besonders über 90 % besitzt.

**20.** Verfahren nach Anspruch 19, **dadurch gekennzeichnet, daß** die Wellenlänge 210 bis 600 nm beträgt, und daß das Lösungsmittel vorzugsweise unter den folgenden Verbindungen ausgewählt ist: Acetonitril; Ethanol; Butanol; Dichlormethan; Cyclohexan; Cyclopentan; 1,2-Dichlorethan; 2,2-Dimethylbutan; n-Heptan; n-Hexan; Methanol; 2-Methylbutan; Iso-Oktan, n-Pentan; 2-Propanol; 1,2,2-Trichlor-trifluorethan; 2,2,2-Trifluorethanol, oder deren Gemischen.

**21.** Verfahren nach irgendeinem der Ansprüche 17 bis 20, **dadurch gekennzeichnet, daß** man eine Temperatur $T_R$ unter oder gleich der Siedetemperatur des gewählten Lösungsmittels bei vorgegebenem Druck vorsieht.

**22.** Verfahren nach Anspruch 21, **dadurch gekennzeichnet, daß** die gewünschten Verbindungen Perhalogen-, insbesondere Perfluoralkyl- und/oder S-Alkylverbindungen sind, und daß $T_R$ gleich oder größer 20 °C, vorzugsweise 35 °C und insbesondere etwa 40 °C ist.

**23.** Verfahren nach Anspruch 21, **dadurch gekennzeichnet, daß** die gewünschten Verbindungen Perhalogen-, insbesondere Perfluoracylverbindungen sind, und daß $T_R$ unter oder gleich 30 °C, vorzugsweise 25 °C und insbesondere bei etwa 20 °C liegt.

**24.** Verfahren nach einem der Ansprüche 14 bis 23, **dadurch gekennzeichnet, daß** das eingesetzte Substrat der folgenden Formel entspricht:

$$R' - S - S - R'' \qquad (IV)$$

in der die Reste R' und R" der Verbindung (IV) und der Rest R der Verbindung (I) einander gleich oder verschieden sind; sie sind vorzugsweise gleich und entsprechen der obigen Definition von R.

**25.** Verfahren nach einem der Ansprüche 14 bis 24, **dadurch gekennzeichnet, daß** das eingesetzte Substrat wenigstens teilweise organisch, vorzugsweise ungesättigt ist, und insbesondere unter den (Cyclo)-alkenen, (Cyclo)alkinen, Aromaten oder deren Gemischen gewählt ist.

**26.** Verfahren nach Anspruch 25, **dadurch gekennzeichnet, daß** das Substrat von wenigstens einem der Produkte gebildet wird, die wenigstens einer der folgenden chemischen Klassen angehören:

- bei der ungesättigten Gruppe nichtfunktionalisierter Alkene und Alkine,
- Enolether und -ester,
- Enolperhalogencarboxylate,
- Vinylsulfide,
- Enamine,
- Enoxy-, Enthio- und Enaminostannane,
- Allystannane,
- Enoxy-, Enthio- und Enaminosilane.

**27.** Verfahren nach einem der Ansprüche 14 bis 26, **dadurch gekennzeichnet, daß** man das Molverhältnis von Thioester (I)/Substrat zwischen 0,1 und 10, vorzugsweise 0,5 und 2 und speziell 0,9 und 1,1 wählt.

**28.** Verfahren nach einem der Ansprüche 14 bis 27, **dadurch gekennzeichnet, daß** die Reaktion in einer Thermolyse besteht.

**29.** Verfahren nach Anspruch 28, **dadurch gekennzeichnet, daß** die Temperatur der Thermolyse zwischen 350 und 650 °C, vorzugsweise 400 und 600 °C, liegt.

**30.** Verfahren nach irgendeinem der Ansprüche 28 und 29, **dadurch gekennzeichnet, daß** die Thermolyse in einem Rohrreaktor aus Glas oder Quartz durchgeführt wird.

**31.** Perfluoralkylverbindung der Formel (VI)

$$(R_4)(R_5)CH — C(R_f)(R_6)(R_7) \qquad (IV)$$

**dadurch gekennzeichnet, daß** sie nach dem Verfahren nach einem der vorangegangenen Ansprüche aus einem Substrat der Formel (V)

$$(R_4)(R_5)C=C(R_6)(R_7)$$

hergestellt werden kann, in der $R_4$, $R_5$, $R_6$, $R_7$ gleich oder verschieden unter Wasserstoff, Kohlenwasserstoffresten, insbesondere den Alkylen einschliesslich den Cycloalkylen und Aralkylen und den Arylen gewählt sind mit der Bedingung, daß wenigstens einer der Reste $R_4$, $R_5$, $R_6$, $R_7$ einen einwertigen Rest mit einem Siliciumatom bedeutet, das von der freien Valenz wenigstens ein, insbesondere wenigstens zwei Kohlenstoffatome entfernt ist.

## Claims

**1.** Reactant, **characterized in that** it comprises, for successive or simultaneous addition:

• a compound of the formula (I):

$$R_f\text{-}M(X)\ (Z)n\text{-}Y\text{-}R;$$

with R denoting a carbon-containing radical, advantageously of at most 15, preferably 10 carbon atoms, preferably chosen from alkyls, aryls, acyls, including hydrocarbylchalcogenylcarbonyls, and thioacyls, including hydrocarbylchalcogenylthiocarbonyl;
with $R_f$ denoting a radical of formula:

$$[R_2\text{-}(C\Xi_2)p\text{-}][R_1\text{-}(C\Xi_2)_m\text{-}]CF\text{-}$$

where $R_1$ and $R_2$, which are similar or different, denote an atom of light halogen, fluorine or chlorine, preferably fluorine, a carbon-containing radical
where m is zero or an integer chosen from the closed range from 0 and 12;
where p is zero or an integer chosen from the closed range from 0 and 12;
where the Ξ, which are similar or different, denote perhalogenated radicals, advantageously perfluorinated radicals, chlorine or preferably fluorine atoms; with the condition that when m and/or p are equal to zero, $R_1$ and/or $R_2$ are electron-withdrawing, advantageously a fluorine or chlorine atom, preferably a fluorine atom,
with n denoting zero or 1;
with M denoting a metalloid chosen from carbon and the chalcogehs of a row higher than oxygen;
with X, Y and Z, which are similar or different, denoting a chalcogen, with the proviso that X, Y and, when n is other than zero, Z are not all oxygen;

- a radical-generator chosen from radicals obtained from the actinic activation or thermolysis of compounds of formula I or obtained from a chemical initiator, advantageously chosen from peroxides, azo derivatives and stannanes.

**2.** Reactant according to Claim 1, **characterized in that** Rf denotes a radical of formula (II):

R1-(CΞ2)m-CF2-

where R1 is a fluorine (or chlorine) atom or a carbon-containing radical;
where m is zero or an integer included between 0 and 12;
where Ξ, which are similar or different, represent chlorine or preferably fluorine atoms;
with the condition that when m is equal to zero, R1 is electron-withdrawing, preferably a fluorine or chlorine atom

**3.** Reactant according to Claims 1 and 2, **characterized in that** the said radical-generator is the compound of formula (I) itself, subjected to an actinic source, advantageously of wavelength included between 200 and 800 nm.

**4.** Reactant according to Claims 1 to 3, **characterized in that** the said radical-generator is another chemical compound giving rise to free radicals, chosen from peroxides, azo derivatives and stannanes.

**5.** Reactant according to Claims 1 to 4, **characterized in that** it additionally comprises a solvent.

**6.** Reactant according to Claims 1 to 5, **characterized in that** Y is chosen from selenium, sulphur and tellurium, advantageously chosen from selenium and sulphur, preferably a sulphur.

**7.** Reactant according to Claims 1 to 6, **characterized in that** X is chosen from oxygen and sulphur, preferably oxygen.

**8.** Reactant according to Claims 1 to 7, **characterized in that** Z is chosen from oxygen and sulphur, preferably oxygen.

**9.** Reactant according to Claims 1 to 8, **characterized in that** M is a carbon atom, n is zero, X is oxygen and Y is sulphur or selenium.

**10.** Reactant according to Claims 1 to 8, **characterized in that** M is a sulphur or selenium atom, n is one, X is oxygen and Y is sulphur or selenium.

**11.** Compound of formula (I)

$$R_f\text{-}M(X)\ (Z)n\text{-}Y\text{-}R;$$

with R denoting a carbon-containing radical, advantageously of at most 15, preferably 10 carbon atoms, chosen

from acyls, including hydrocarbylchalcogenylcarbonyls, and thioacyls, including hydrocarbylchalcogenylthiocarbonyls;
with $R_f$ denoting a radical of formula:

$$[R_2\text{-}(C\Xi_2)p\text{-}][R_1\text{-}(C\Xi_2)_m\text{-}]CF\text{-}$$

where $R_1$ and $R_2$, which are similar or different, denote an atom of light halogen, fluorine or chlorine, preferably fluorine, a carbon-containing radical
where m is zero or an integer chosen from the closed range from 0 and 12;
where p is zero or an integer chosen from the closed range from 0 and 12;
where the Ξ, which are similar or different, denote perhalogenated radicals, advantageously perfluorinated radicals, chlorine or preferably fluorine atoms; with the condition that when m and/or p are equal to zero, $R_1$ and/or $R_2$ are electron-withdrawing, advantageously a fluorine or chlorine atom, preferably a fluorine atom,
with n denoting zero or 1;
with M denoting a metalloid chosen from carbon and the chalcogens of a row higher than oxygen;
with Y denoting a sulphur or a selenium,
with X and Z, which are similar or different, denoting a chalcogen.

**12.** Compound of formula (I)

$$R_f\text{-}M(X)\ (Z)n\text{-}Y\text{-}R;$$

with R denoting a carbon-containing radical, advantageously of at most 15, preferably 10 carbon atoms, preferably chosen from alkyls, aryls, acyls, including hydrocarbylchalcogenylcarbonyls, and
thioacyls, including hydrocarbylchalcogenylthiocarbonyl;
with $R_f$ denoting a radical of formula:

$$[R_2\text{-}\ (C\Xi_2)_p\text{-}]\ [R_1\text{-}\ (C\Xi_2)_m\text{-}]CF\text{-}$$

where $R_1$ and $R_2$, which are similar or different, denote an atom of light halogen, fluorine or chlorine, preferably fluorine, a carbon-containing radical
where m is zero or an integer chosen from the closed range from 0 and 12;
where p is zero or an integer chosen from the closed range from 0 and 12;
where the Ξ, which are similar or different, denote perhalogenated radicals, advantageously perfluorinated radicals, chlorine or preferably fluorine atoms; with the condition that when m and/or p are equal to zero, $R_1$ and/or $R_2$ are electron-withdrawing, advantageously a fluorine or chlorine atom, preferably a fluorine atom,
with n denoting zero or 1;
with M denoting a metalloid chosen from carbon and the chalcogens of a row higher than oxygen;
with X, Y and Z, which are similar or different, denoting a chalcogen;

**characterized in that** $R_f$ contains more than three carbon.

**13.** Compound according to Claims 11 and 12, **characterized in that** $R_f$ is a carbon-containing radical of at most 20 carbons, preferably of at most 15 carbons.

**14.** Process for the treatment of a compound which has at least one nucleophilic functional group, **characterized in that** it comprises at least one stage in which the said compound which has at least one nucleophilic functional group is placed in contact with a compound of formula (I):

$$R_f\text{-}M(X)\ (Z)n\text{-}Y\text{-}R;$$

with R denoting a carbon-containing radical, advantageously of at most 15, preferably 10 carbon atoms, preferably chosen from alkyls, aryls acyls and thioacyls;
with $R_f$ denoting a radical of formula:

$$[R_2\text{-}(C\Xi_2)_p\text{-}]\ [R_1\text{-}(C\Xi_2)_m\text{-}]CF\text{-}$$

where $R_1$ and $R_2$, which are similar or different, denote an atom of light halogen, fluorine or chlorine, preferably fluorine, a carbon-containing radical
where m is zero or an integer chosen from the closed range from 0 and 12;
where p is zero or an integer chosen from the closed range from 0 and 12;
where the Ξ, which are similar or different, denote perhalogenated radicals, advantageously perfluorinated radicals, chlorine or preferably fluorine atoms; with the condition that when m and/or p are equal to zero, $R_1$ and/or $R_2$ are electron-withdrawing, advantageously a fluorine or chlorine atom, preferably a fluorine atom, with n denoting zero or 1;
with M denoting a metalloid chosen from carbon and the chalcogens of a row higher than oxygen;
with X, Y and Z which are identical or different, denoting a chalcogen, with the condition that X, Y and Z are not all oxygen when m is other than zero; the said placing in contact being performed with radical initiation by means of an actinic source or of a chemical radical initiator.

**15.** Process according to Claim 14, **characterized in that** a radical reaction of conversion of compound I **(I)** to perhaloalkylated thioether is used in the absence of substrate.

**16.** Process according to Claim 14, **characterized in that** it consists essentially in reacting compound **(I)** by a radical route with at least one substrate.

**17.** Process according to either of Claims 14 and 16, **characterized in that** the reaction consists of a photolysis of a compound **(I)** in the presence of a substrate in a reaction mixture optionally including an organic solvent, at a wavelength corresponding to the maximum absorption of the chromophore group.

**18.** Process according to Claim 17, **characterized in that** a choice is made between 200 and 800 nm, preferably between 210 and 600 nm.

**19.** Process according to Claims 17 and 18, **characterized in that** the solvent is chosen so that it has a transparency higher than or equal to 50 %, preferably to 70 % and, still more preferably, to 90 % at the wavelength.

**20.** Process according to Claim 19, **characterized in that** the wavelength is of the order of 210-600 nm and **in that** the solvent is selected preferably from the following compounds: acetonitrile, ethanol, butanol, dichloromethane, cyclohexane, cyclopentane, 1,2-dichloroethane, 2,2-dimethylbutane, n-heptane, n-hexane, methanol, 2-methylbutane, isooctane, n-pentane, 2-propanol, 1,2,2-trichlorotrifluoroethane, 2,2,2-trifluoroethanol or a mixture of these.

**21.** Process according to any one of Claims 17 to 20, **characterized in that** a temperature $T_R$ is provided which is lower than or equal to the boiling temperature, at given pressure, of the selected solvent.

**22.** Process according to Claim 21, **characterized in that** the compounds aimed at are perhalo, in particular perfluoroalkylated and/or -S-alkylated, compounds and **in that** TR is higher than or equal to 20°C, preferably to 35°C and, still more preferably, of the order of 40°C.

**23.** Process according to Claim 21, **characterized in that** the compounds aimed at are perhalo, in particular perfluoroacylated, compounds and **in that** $T_R$ is lower than or equal to 30°C, preferably to 25°C and, still more preferably, is of the order of 20°C.

**24.** Process according to any one of Claims 14 to 23, **characterized in that** the substrate used corresponds to the following formula:

$$R'\text{-}S\text{-}S\text{-}R'' \qquad (IV)$$

where the radicals $R^1$, $R^{11}$ of compound (IV) and radical R of compound (I) may be identical to or different from each other; they are preferably identical and correspond to the definition of R given above.

**25.** Process according to one of Claims 14 to 11, **characterized in that** the substrate used is at least partially organic,

preferably unsaturated and, still more preferably, selected from (cyclo)alkenes, (cyclo)alkynes, aromatics or their mixtures.

**26.** Process according to Claim 25, **characterized in that** the substrate is formed by at least one of the products belonging to at least one of the following chemical classes:

- alkenes and alkynes which are not functionalized under unsaturation,
- ethers and enol esters,
- enol perhalocarboxylates,
- vinyl sulphides,
- enamines,
- enoxy, enethio and enaminostannanes,
- allylstannanes,
- enoxy, enethio and enaminosilanes,
- allylsilanes.

**27.** Process according to any one of Claims 14 to 26, **characterized in that** the thioloester **(I)**/substrate molar ratio is chosen to be between 0.1 and 10, preferably between 0.5 and 2 and still more preferably between 0.9 and 1.1.

**28.** Process according to any one of Claims 14 to 27, **characterized in that** the reaction consists of a thermolysis.

**29.** Process according to Claim 28, **characterized in that** the temperature of thermolysis is between 350 and 650°C, preferably between 400 and 600°C.

**30.** Process according to either of Claims 28 and 29, **characterized in that** the thermolysis is carried out in a tubular reactor made of glass or quartz.

**31.** Perfluoroalkyl derivative of formula VI:

$$(R_4)\ (R_5)CH\text{-}C(\mathbf{R_f})\ (R_6)\ (R_7)\ (VI)$$

**characterized in that** it is obtainable by the process according to one of the preceding claims, from a substrate of formula (V)

$$(R_4)\ (R_5)C{=}C(R_6)\ (R_7)$$

with $R_4$, $R_5$, $R_6$ and $R_7$, which are similar or different, are chosen from hydrogen, hydrocarbon radicals and, in particular, alkyls, including cycloalkyls and aralkyls, and from aryls, with the condition that at least one of $R_4$, $R_5$, $R_6$ and $R_7$ denotes a monovalent radical bearing a silicon atom at a distance from the free valency of at least one, advantageously of at least two, carbon atoms.